# EUROPÄISCHE PATENTSCHRIFT

(11) **EP 1 259 622 B1**
(45) Veröffentlichungstag und Bekanntmachung des Hinweises auf die Patenterteilung: **28.09.2005**
(21) Anmeldenummer: 01929377.8
(22) Anmeldetag: 01.03.2001
(51) Int. Cl.: C12N 15/54, C12N 15/55, C12N 9/16, C12N 9/10, C12N 1/21, C12Q 1/68, C12N 15/10, C12P 13/06

(54) **NUKLEOTIDSEQUENZEN KODIEREND FUR PROTEINE BETEILIGT AN DER BIOSYNTHESE VON L-SERIN, VERBESSERTES VERFAHREN ZUR MIKROBIELLEN HERSTELLUNG VON L-SERIN SOWIE EIN DAZU GEEIGNETER GENETISCH VERANDERTER MIKROORGANISMUS**
NUCLEOTIDE SEQUENCES ENCODING PROTEINS THAT TAKE PART IN THE BIOSYNTHESIS OF L-SERINE, IMPROVED METHOD FOR MICROBIALLY PRODUCING L-SERINE, AND GENETICALLY MODIFIED MICROORGANISM SUITABLE FOR USE IN SAID METHOD
SEQUENCES NUCLEOTIDIQUES CODANT POUR DES PROTEINES PARTICIPANT A LA BIOSYNTHESE DE LA L-SERINE, METHODE AMELIOREE DE PRODUCTION MICROBIENNE DE L-SERINE ET MICRO-ORGANISME GENETIQUEMENT MODIFIE EN VUE DE LADITE PRODUCTION

(30) Priorität: 01.03.2000 DE 10009799; 11.09.2000 DE 10044831
(43) Veröffentlichungstag der Anmeldung: 27.11.2002
(73) Patentinhaber: FORSCHUNGSZENTRUM JÜLICH GMBH, 52425 Jülich (DE)
(72) Erfinder: ZIEGLER, Petra, Cambridge CB5 8HJ (GB); EGGELING, Lothar, 52428 Jülich (DE); SAHM, Hermann, 52428 Jülich (DE); PETERS-WENDISCH, Petra, 52428 Jülich (DE)
(74) Vertreter: Fitzner, Ulrich
(86) Internationale Anmeldenummer: PCT/EP2001/002283
(87) Internationale Veröffentlichungsnummer: WO 2001/064899

(56) Entgegenhaltungen:
- EP-A- 0 931 833
- EP-A- 1 108 790
- WO-A-01/00843
- US-A- 5 380 657
- BERGER S AND KIMMEL A: "Methods in enzymolgy Volume 152" 1988 , ACADEMIC PRESS , SAN DIEGO XP002183713 Seite 516, letzter Absatz -Seite 517, Absatz 1
- DATABASE WPI Section Ch, Week 199249 Derwent Publications Ltd., London, GB; Class B05, AN 1992-405386 XP002183715 & SU 1 412 288 A (IND MICROORGANISMS GENETICS SELECTION), 7. Dezember 1991 (1991-12-07)
- DATABASE WPI Section Ch, Week 199431 Derwent Publications Ltd., London, GB; Class B04, AN 1994-251695 XP002183716 & JP 06 181776 A (KYOWA HAKKO KOGYO KK), 5. Juli 1994 (1994-07-05)
- LEHNINGER A: "Biochemistry " 1972 , WORTH PUBLISHERS INC , NEW YORK XP002183714 Abbildungen 24-2,24-6,24-17

### SEQUENCE LISTING

<110> Forschungszentrum Jülich GmbH
<120> Nukleotidsequenzen kodierend für Proteine beteiligt an der Biosynthese von L-Serin, verbessertes Verfahren zur mikrobiellen Herstellung von L-Serin sowie ein dazu geeigneter genetisch veränderter Mikr
<130> FZJ-9912-PCT
<140>
   <141>
<160> 12
<170> PatentIn Ver. 2.1
<210> 1
   <211> 1765
   <212> DNA
   <213> C. glutamicum ATCC 14 752
<220>
   <221> CDS
   <222> (414) .. (1613)
   <223> ser B (Phosphoserin-phosphatase)
<400> 1
   atgattgaac gcatgcgcgc agaggcacac actggacatc acgatgatat taatgctcca 60
   gaattgggta ccgccccagc ccttgcatct gactccagcc gctaaaagcg tctgatttaa 120
   gtcggtacct gactaaataa gcaccagccc agcagagata ttctgccggg gctggtgctt 180
   ttcatattcc gacttggggc acccctgaat acatctcacc caattcccca taactagaca 240
   attgcccagc aacgactgat aagtctccaa tgtcgtgttc cgcgctcaga catgagacaa 300
   ttgttgccgt gactgaactc atccagaatg aatcccaaga aatcgctgag ctggaagccg 360
   gccagcaggt tgcattgcgt gaaggttatc gttatcttcc tgcggtgatc aca gtg    416
                                                               Met
                                                                1
   agc ggt aaa gac cgc cca ggt gtg act gcc gcg ttc ttt agg gtc ttg    464
   Ser Gly Lys Asp Arg Pro Gly Val Thr Ala Ala Phe Phe Arg Val Leu
               5                   10                  15
   tcc gct aat cag gtt cag gtc ttg gac gtt gag cag tca atg ttc cgt    512
   Ser Ala Asn Gln Val Gln Val Leu Asp Val Glu Gln Ser Met Phe Arg
           20                  25                  30
   ggc ttt ttg aac ttg gcg gcg ttt gtg ggt atc gca cct gag cgt gtc    560
   Gly Phe Leu Asn Leu Ala Ala Phe Val Gly Ile Ala Pro Glu Arg Val
       35                  40                  45
   gag acc gtc acc aca ggc ctg act gac acc ctc aag gtg cat gga cag    608
   Glu Thr Val Thr Thr Gly Leu Thr Asp Thr Leu Lys Val His Gly Gln
   50                  55                  60                  65
   tcc gtg gtg gtg gag ctg cag gaa act gtg cag tcg tcc cgt cct cgt    656
   Ser Val Val Val Glu Leu Gln Glu Thr Val Gln Ser Ser Arg Pro Arg
                   70                  75                  80
   tct tcc cat gtt gtt gtg gtg ttg ggt gat ccg gtt gat gcg ttg gat    704
   Ser Ser His Val Val Val Val Leu Gly Asp Pro Val Asp Ala Leu Asp
               85                  90                  95
   att tcc cgc att ggt cag acc ctg gcg gat tac gat gcc aac att gac    752
   Ile Ser Arg Ile Gly Gln Thr Leu Ala Asp Tyr Asp Ala Asn Ile Asp
           100                 105                 110
   acc att cgt ggt att tcg gat tac cct gtg acc ggc ctg gag ctg aag    800
   Thr Ile Arg Gly Ile Ser Asp Tyr Pro Val Thr Gly Leu Glu Leu Lys
       115                 120                 125
   gtg act gtg ccg gat gtc agc cct ggt ggt ggt gaa gcg atg cgt aag    848
   Val Thr Val Pro Asp Val Ser Pro Gly Gly Gly Glu Ala Met Arg Lys
   130                 135                 140                 145
   gcg ctt gct gct ctt acc tct gag ctg aat gtg gat att gcg att gag    896
   Ala Leu Ala Ala Leu Thr Ser Glu Leu Asn Val Asp Ile Ala Ile Glu
                   150                 155                 160
   cgt tct ggt ttg ctg cgt cgt tct aag cgt ctg gtg tgc ttc gat tgt    944
   Arg Ser Gly Leu Leu Arg Arg Ser Lys Arg Leu Val Cys Phe Asp Cys
               165                 170                 175
   gat tcc acg ttg atc act ggt gag gtc att gag atg ttg gcg gct cac    992
   Asp Ser Thr Leu Ile Thr Gly Glu Val Ile Glu Met Leu Ala Ala His
           180                 185                 190
   gcg ggc aag gaa gct gaa gtt gcg gca gtt act gag cgt gcg atg cgc    1040
   Ala Gly Lys Glu Ala Glu Val Ala Ala Val Thr Glu Arg Ala Met Arg
       195                 200                 205
   ggt gag ctc gat ttc gag gag tct ctg cgt gag cgt gtg aag gcg ttg    1088
   Gly Glu Leu Asp Phe Glu Glu Ser Leu Arg Glu Arg Val Lys Ala Leu
   210                 215                 220                 225
   gct ggt ttg gat gcg tcg gtg atc gat gag gtc gct gcc gct att gag    1136
   Ala Gly Leu Asp Ala Ser Val Ile Asp Glu Val Ala Ala Ala Ile Glu
                   230                 235                 240
   ctg acc cct ggt gcg cgc acc acg atc cgt acg ctg aac cgc atg ggt    1184
   Leu Thr Pro Gly Ala Arg Thr Thr Ile Arg Thr Leu Asn Arg Met Gly
               245                 250                 255
   tac cag acc gct gtt gtt tcc ggt ggt ttc atc cag gtg ttg gaa ggt    1232
   Tyr Gln Thr Ala Val Val Ser Gly Gly Phe Ile Gln Val Leu Glu Gly
           260                 265                 270
   ttg gct gag gag ttg gag ttg gat tat gtc cgc gcc aac act ttg gaa    1280
   Leu Ala Glu Glu Leu Glu Leu Asp Tyr Val Arg Ala Asn Thr Leu Glu
       275                 280                 285
   atc gtt gat ggc aag ctg acc ggc aac gtc acc ggc aag atc gtt gac    1328
   Ile Val Asp Gly Lys Leu Thr Gly Asn Val Thr Gly Lys Ile Val Asp
   290                 295                 300                 305
   cgc gct gcg aag gct gag ttc ctc cgt gag ttc gct gcg gat tct ggg   1376
   Arg Ala Ala Lys Ala Glu Phe Leu Arg Glu Phe Ala Ala Asp Ser Gly
                   310                 315                 320
   ctg aag atg tac cag act gtc gct gtc ggt gat ggc gct aat gac atc   1424
   Leu Lys Met Tyr Gln Thr Val Ala Val Gly Asp Gly Ala Asn Asp Ile
               325                 330                 335
   gat atg ctc tcc gct gcg ggt ctg ggt gtt gct ttc aac gcg aag cct   1472
   Asp Met Leu Ser Ala Ala Gly Leu Gly Val Ala Phe Asn Ala Lys Pro
           340                 345                 350
   gcg ctg aag gag att gcg gat act tcc gtg aac cac cca ttc ctc gac   1520
   Ala Leu Lys Glu Ile Ala Asp Thr Ser Val Asn His Pro Phe Leu Asp
       355                 360                 365
   gag gtt ttg cac atc atg ggc att tcc cgc gac gag atc gat ctg gcg   1568
   Glu Val Leu His Ile Met Gly Ile Ser Arg Asp Glu Ile Asp Leu Ala
   370                 375                 380                 385
   gat cag gaa gac ggc acc ttc cac cgc gtt cca ttg aca aat gcc       1613
   Asp Gln Glu Asp Gly Thr Phe His Arg Val Pro Leu Thr Asn Ala
                   390                 395                 400
   taaagattcg cttctcgacg cccacctcct cctcaaggcc cgggctagcg acgggccaca 1673
   tagcgaggat ccttcggatc cttcgaccgt tcaggcaatg cagatcgcgt tgcaacttcc 1733
   gaaacagaat ccgccccggc ggacagatgt gc                               1765
<210> 2
   <211> 400
   <212> PRT
   <213> C. glutamicum ATCC 14 752
<400> 2
   Met Ser Gly Lys Asp Arg Pro Gly Val Thr Ala Ala Phe Phe Arg Val
    1               5                  10                  15
   Leu Ser Ala Asn Gln Val Gln Val Leu Asp Val Glu Gln Ser Met Phe
               20                  25                  30
   Arg Gly Phe Leu Asn Leu Ala Ala Phe Val Gly Ile Ala Pro Glu Arg
           35                  40                  45
   Val Glu Thr Val Thr Thr Gly Leu Thr Asp Thr Leu Lys Val His Gly
       50                  55                  60
   Gln Ser Val Val Val Glu Leu Gln Glu Thr Val Gln Ser Ser Arg Pro
   65                  70                  75                  80
   Arg Ser Ser His Val Val Val Val Leu Gly Asp Pro Val Asp Ala Leu
                   85                  90                  95
   Asp Ile Ser Arg Ile Gly Gln Thr Leu Ala Asp Tyr Asp Ala Asn Ile
               100                 105                 110
   Asp Thr Ile Arg Gly Ile Ser Asp Tyr Pro Val Thr Gly Leu Glu Leu
           115                 120                 125
   Lys Val Thr Val Pro Asp Val Ser Pro Gly Gly Gly Glu Ala Met Arg
       130                 135                 140
   Lys Ala Leu Ala Ala Leu Thr Ser Glu Leu Asn Val Asp Ile Ala Ile
   145                 150                 155                 160
   Glu Arg Ser Gly Leu Leu Arg Arg Ser Lys Arg Leu Val Cys Phe Asp
                   165                 170                 175
   Cys Asp Ser Thr Leu Ile Thr Gly Glu Val Ile Glu Met Leu Ala Ala
               180                 185                 190
   His Ala Gly Lys Glu Ala Glu Val Ala Ala Val Thr Glu Arg Ala Met
           195                 200                 205
   Arg Gly Glu Leu Asp Phe Glu Glu Ser Leu Arg Glu Arg Val Lys Ala
       210                 215                 220
   Leu Ala Gly Leu Asp Ala Ser Val Ile Asp Glu Val Ala Ala Ala Ile
   225                 230                 235                 240
   Glu Leu Thr Pro Gly Ala Arg Thr Thr Ile Arg Thr Leu Asn Arg Met
                   245                 250                 255
   Gly Tyr Gln Thr Ala Val Val Ser Gly Gly Phe Ile Gln Val Leu Glu
               260                 265                 270
   Gly Leu Ala Glu Glu Leu Glu Leu Asp Tyr Val Arg Ala Asn Thr Leu
           275                 280                 285
   Glu Ile Val Asp Gly Lys Leu Thr Gly Asn Val Thr Gly Lys Ile Val
       290                 295                 300
   Asp Arg Ala Ala Lys Ala Glu Phe Leu Arg Glu Phe Ala Ala Asp Ser
   305                 310                 315                 320
   Gly Leu Lys Met Tyr Gln Thr Val Ala Val Gly Asp Gly Ala Asn Asp
                   325                 330                 335
   Ile Asp Met Leu Ser Ala Ala Gly Leu Gly Val Ala Phe Asn Ala Lys
               340                 345                 350
   Pro Ala Leu Lys Glu Ile Ala Asp Thr Ser Val Asn His Pro Phe Leu
           355                 360                 365
   Asp Glu Val Leu His Ile Met Gly Ile Ser Arg Asp Glu Ile Asp Leu
       370                 375                 380
   Ala Asp Gln Glu Asp Gly Thr Phe His Arg Val Pro Leu Thr Asn Ala
   385                 390                 395                 400
<210> 3
   <211> 1469
   <212> DNA
   <213> C. glutamicum ATCC 14 752
<220>
   <221> CDS
   <222> (246) .. (1373)
   <223> ser C (Phosphoserin-phosphatase)
<400> 3
   ttaacaacct agaaattgaa aactttgcaa aactttgagc tacccccaaa ttggtggctg 60
   gtcaactaat ccccgcgttt tcaatagttc ggtgtcgcca gttttgggcg tttttcatcg 120
   tttgggagac tgcgtgaaga atctagggtg ctaggaactg acagcttcag ggttatagtt 180
   gttgggtcag atcgttaacg atccctggcc cttttacttc caagcgcaga aagttgcccg 240
   aagac atg acc gac ttc ccc acc ctg ccc tct gag ttc atc cct ggc gac 290
   Met Thr Asp Phe Pro Thr Leu Pro Ser Glu Phe Ile Pro Gly Asp
   1 5 10 15
   ggc cgt ttc ggc tgc gga cct tcc aag gtt cga cca gaa cag att cag 338
   Gly Arg Phe Gly Cys Gly Pro Ser Lys Val Arg Pro Glu Gln Ile Gln
   20 25 30
   gct att gtc gac gga tcc gca tcc gtc atc ggt acc tca cac cgt cag 386
   Ala Ile Val Asp Gly Ser Ala Ser Val Ile Gly Thr Ser His Arg Gln
   35 40 45
   ccg gca gta aaa aac gtc gtg ggt tca atc cgc gag gga ctc tcc gac 434
   Pro Ala Val Lys Asn Val Val Gly Ser Ile Arg Glu Gly Leu Ser Asp
   50 55 60
   ctc ttc tcc ctt cca gaa ggc tac gag atc atc ctt tcc cta ggt ggt 482
   Leu Phe Ser Leu Pro Glu Gly Tyr Glu Ile Ile Leu Ser Leu Gly Gly
   65 70 75
   gcg acc gca ttc tgg gat gca gca acc ttc gga ctc att gaa aag aag 530
   Ala Thr Ala Phe Trp Asp Ala Ala Thr Phe Gly Leu Ile Glu Lys Lys
   80 85 90 95
   tcc ggt cac ctt tct ttc ggt gag ttc tcc tcc aag ttc gca aag gct 578
   Ser Gly His Leu Ser Phe Gly Glu Phe Ser Ser Lys Phe Ala Lys Ala
   100 105 110
   tct aag ctt gct cct tgg ctc gac gag cca gag atc gtc acc gca gaa 626
   Ser Lys Leu Ala Pro Trp Leu Asp Glu Pro Glu Ile Val Thr Ala Glu
   115 120 125
   acc ggt gac tct ccg gcc cca cag gca ttc gaa ggc gcc gat gtt att 674
   Thr Gly Asp Ser Pro Ala Pro Gln Ala Phe Glu Gly Ala Asp Val Ile
   130 135 140
   gca tgg gca cac aac gaa acc tcc act ggc gcc atg gtt cca gtt ctt 722
   Ala Trp Ala His Asn Glu Thr Ser Thr Gly Ala Met Val Pro Val Leu
   145 150 155
   cgc ccc gaa ggc tct gaa ggc tcc ctg gtt gcc att gac gca acc tcc 770
   Arg Pro Glu Gly Ser Glu Gly Ser Leu Val Ala Ile Asp Ala Thr Ser
   160 165 170 175
   ggc gct ggt gga ctg cca gta gac atc aag aac tcc gat gtt tac tac 818
   Gly Ala Gly Gly Leu Pro Val Asp Ile Lys Asn Ser Asp Val Tyr Tyr
                   180                 185                 190
   ttc tcc cca cag aag tgc ttc gca tcc gac ggt ggc ctg tgg ctt gca      866
   Phe Ser Pro Gln Lys Cys Phe Ala Ser Asp Gly Gly Leu Trp Leu Ala
               195                 200                 205
   gcg atg agc cca gca gct ctc gag cgc atc gag aag atc aac gct tcc      914
   Ala Met Ser Pro Ala Ala Leu Glu Arg Ile Glu Lys Ile Asn Ala Ser
           210                 215                 220
   gat cgc ttc atc cct gag ttc ctc aac ctg cag acc gca gtg gat aac      962
   Asp Arg Phe Ile Pro Glu Phe Leu Asn Leu Gln Thr Ala Val Asp Asn
       225                 230                 235
   tcc ctg aag aac cag acc tac aac acc cca gct gtt gct acc ttg ctg     1010
   Ser Leu Lys Asn Gln Thr Tyr Asn Thr Pro Ala Val Ala Thr Leu Leu
   240                 245                 250                 255
   atg ctg gac aac cag gtc aag tgg atg aac tcc aac ggc ggc ctg gat     1058
   Met Leu Asp Asn Gln Val Lys Trp Met Asn Ser Asn Gly Gly Leu Asp
                   260                 265                 270
   gga atg gtt gct cgc acc aca gca agc tcc tcc gcc ctg tac aac tgg     1106
   Gly Met Val Ala Arg Thr Thr Ala Ser Ser Ser Ala Leu Tyr Asn Trp
               275                 280                 285
   gct gag gct cgc gag gag gca tcc cca tac gtg gca gat gca gct aag     1154
   Ala Glu Ala Arg Glu Glu Ala Ser Pro Tyr Val Ala Asp Ala Ala Lys
           290                 295                 300
   cgc tcc ctc gtt gtc ggc acc atc gac ttc gat gac tcc atc gac gca     1202
   Arg Ser Leu Val Val Gly Thr Ile Asp Phe Asp Asp Ser Ile Asp Ala
       305                 310                 315
   gca gtg atc gct aag ata ctg cgc gca aac ggc atc ctg gac acc gag     1250
   Ala Val Ile Ala Lys Ile Leu Arg Ala Asn Gly Ile Leu Asp Thr Glu
   320                 325                 330                 335
   cct tac cgc aag ctg gga cgc aac cag ctg cgc atc ggt atg ttc cca     1298
   Pro Tyr Arg Lys Leu Gly Arg Asn Gln Leu Arg Ile Gly Met Phe Pro
                   340                 345                 350
   gcg atc gat tcc acc gat gtg gaa aag ctc acc gga gca atc gac ttc     1346
   Ala Ile Asp Ser Thr Asp Val Glu Lys Leu Thr Gly Ala Ile Asp Phe
               355                 360                 365
   atc ctc gat ggc ggt ttt gca agg aag taataccccc actttgaaaa          1393
   Ile Leu Asp Gly Gly Phe Ala Arg Lys
           370                 375
   acaccccgta cagttacacc agtacggggt gttttttagt taagcttggg tggtgttttt 1453
   tagttaagct tgggtg                                                    1469
<210> 4
   <211> 376
   <212> PRT
   <213> C. glutamicum ATCC 14 752
<400> 4
   Met Thr Asp Phe Pro Thr Leu Pro Ser Glu Phe Ile Pro Gly Asp Gly
    1               5                  10                  15
   Arg Phe Gly Cys Gly Pro Ser Lys Val Arg Pro Glu Gln Ile Gln Ala
               20                  25                  30
   Ile Val Asp Gly Ser Ala Ser Val Ile Gly Thr Ser His Arg Gln Pro
           35                  40                  45
   Ala Val Lys Asn Val Val Gly Ser Ile Arg Glu Gly Leu Ser Asp Leu
       50                  55                  60
   Phe Ser Leu Pro Glu Gly Tyr Glu Ile Ile Leu Ser Leu Gly Gly Ala
   65                  70                  75                  80
   Thr Ala Phe Trp Asp Ala Ala Thr Phe Gly Leu Ile Glu Lys Lys Ser
                   85                  90                  95
   Gly His Leu Ser Phe Gly Glu Phe Ser Ser Lys Phe Ala Lys Ala Ser
               100                 105                 110
   Lys Leu Ala Pro Trp Leu Asp Glu Pro Glu Ile Val Thr Ala Glu Thr
           115                 120                 125
   Gly Asp Ser Pro Ala Pro Gln Ala Phe Glu Gly Ala Asp Val Ile Ala
       130                 135                 140
   Trp Ala His Asn Glu Thr Ser Thr Gly Ala Met Val Pro Val Leu Arg
   145                 150                 155                 160
   Pro Glu Gly Ser Glu Gly Ser Leu Val Ala Ile Asp Ala Thr Ser Gly
                   165                 170                 175
   Ala Gly Gly Leu Pro Val Asp Ile Lys Asn Ser Asp Val Tyr Tyr Phe
               180                 185                 190
   Ser Pro Gln Lys Cys Phe Ala Ser Asp Gly Gly Leu Trp Leu Ala Ala
           195                 200                 205
   Met Ser Pro Ala Ala Leu Glu Arg Ile Glu Lys Ile Asn Ala Ser Asp
       210                 215                 220
   Arg Phe Ile Pro Glu Phe Leu Asn Leu Gln Thr Ala Val Asp Asn Ser
   225                 230                 235                 240
   Leu Lys Asn Gln Thr Tyr Asn Thr Pro Ala Val Ala Thr Leu Leu Met
                   245                 250                 255
   Leu Asp Asn Gln Val Lys Trp Met Asn Ser Asn Gly Gly Leu Asp Gly
               260                 265                 270
   Met Val Ala Arg Thr Thr Ala Ser Ser Ser Ala Leu Tyr Asn Trp Ala
           275                 280                 285
   Glu Ala Arg Glu Glu Ala Ser Pro Tyr Val Ala Asp Ala Ala Lys Arg
       290                 295                 300
   Ser Leu Val Val Gly Thr Ile Asp Phe Asp Asp Ser Ile Asp Ala Ala
   305                 310                 315                 320
   Val Ile Ala Lys Ile Leu Arg Ala Asn Gly Ile Leu Asp Thr Glu Pro
   325 330 335
   Tyr Arg Lys Leu Gly Arg Asn Gln Leu Arg Ile Gly Met Phe Pro Ala
   340 345 350
   Ile Asp Ser Thr Asp Val Glu Lys Leu Thr Gly Ala Ile Asp Phe Ile
   355 360 365
   Leu Asp Gly Gly Phe Ala Arg Lys
   370 375
<210> 5
   <211> 1627
   <212> DNA
   <213> C. glutanicum ATCC 13 032
<220>
   <221> CDS
   <222> (382) .. (1590)
   <223> ser B (Phosphoserin-phosphatase)
<400> 5
   gcagaggcac acactggaca tcacgatgat attaatgctc cagaattggg taccgcccca 60
   gcccttgcat ctgactccag ccgctaaaag cgtctgattt aagtcggtac ctgactaaat 120
   aagcaccagc cccagcagag ataatctgcc ggggctggtg cttttcatat tccgacttgg 180
   ggcacccctg aatacatctc acccaattcc ccataactag acaattgccc agcaacgact 240
   gataagtctc caatgtcgtg ttccgcgctc agacatgaga caattgttgc cgtgactgaa 300
   ctcatccaga atgaatccca agaaatcgct gagctggaag ccggccagca ggttgcattg 360
   cgtgaaggtt atcttcctgc g gtg atc aca gtg agc ggt aaa gac cgc cca    411
                           Met Ile Thr Val Ser Gly Lys Asp Arg Pro
                            1               5                   10
   ggt gtg act gcc gcg ttc ttt agg gtc ttg tcc gct aat cag gtt cag    459
   Gly Val Thr Ala Ala Phe Phe Arg Val Leu Ser Ala Asn Gln Val Gln
                   15                  20                  25
   gtc ttg gac gtt gag cag tca atg ttc cgt ggc ttt ttg aac ttg gcg    507
   Val Leu Asp Val Glu Gln Ser Met Phe Arg Gly Phe Leu Asn Leu Ala
               30                  35                  40
   gcg ttt gtg ggt atc gca cct gag cgt gtc gag acc gtc acc aca ggc    555
   Ala Phe Val Gly Ile Ala Pro Glu Arg Val Glu Thr Val Thr Thr Gly
           45                  50                  55
   ctg act gac acc ctc aag gtg cat gga cag tcc gtg gtg gtg gag ctg    603
   Leu Thr Asp Thr Leu Lys Val His Gly Gln Ser Val Val Val Glu Leu
       60                  65                  70
   cag gaa act gtg cag tcg tcc cgt cct cgt tct tcc cat gtt gtt gtg    651
   Gln Glu Thr Val Gln Ser Ser Arg Pro Arg Ser Ser His Val Val Val
   75                  80                  85                  90
   gtg ttg ggt gat ccg gtt gat gcg ttg gat att tcc cgc att ggt cag    699
   Val Leu Gly Asp Pro Val Asp Ala Leu Asp Ile Ser Arg Ile Gly Gln
                   95                  100                 105
   acc ctg gcg gat tac gat gcc aac att gac acc att cgt ggt att tcg    747
   Thr Leu Ala Asp Tyr Asp Ala Asn Ile Asp Thr Ile Arg Gly Ile Ser
               110                 115                 120
   gat tac cct gtg acc ggc ctg gag ctg aag gtg act gtg ccg gat gtc    795
   Asp Tyr Pro Val Thr Gly Leu Glu Leu Lys Val Thr Val Pro Asp Val
           125                 130                 135
   agc cct ggt ggt ggt gaa gcg atg cgt aag gcg ctt gct gct ctt acc    843
   Ser Pro Gly Gly Gly Glu Ala Met Arg Lys Ala Leu Ala Ala Leu Thr
       140                 145                 150
   tct gag ctg aat gtg gat att gcg att gag cgt tct ggt ttg ctg cgt    891
   Ser Glu Leu Asn Val Asp Ile Ala Ile Glu Arg Ser Gly Leu Leu Arg
   155                 160                 165                 170
   cgt tct aag cgt ctg gtg tgc ttc gat tgt gat tcc acg ttg atc act    939
   Arg Ser Lys Arg Leu Val Cys Phe Asp Cys Asp Ser Thr Leu Ile Thr
                   175                 180                 185
   ggt gag gtc att gag atg ctg gcg gct cac gcg ggc aag gaa gct gaa    987
   Gly Glu Val Ile Glu Met Leu Ala Ala His Ala Gly Lys Glu Ala Glu
               190                 195                 200
   gtt gcg gca gtt act gag cgt gcg atg cgc ggt gag ctc gat ttc gag    1035
   Val Ala Ala Val Thr Glu Arg Ala Met Arg Gly Glu Leu Asp Phe Glu
           205                 210                 215
   gag tct ctg cgt gag cgt gtg aag gcg ttg gct ggt ttg gat gcg tcg    1083
   Glu Ser Leu Arg Glu Arg Val Lys Ala Leu Ala Gly Leu Asp Ala Ser
       220                 225                 230
   gtg atc gat gag gtc gct gcc gct att gag ctg acc cct ggt gcg cgc    1131
   Val Ile Asp Glu Val Ala Ala Ala Ile Glu Leu Thr Pro Gly Ala Arg
   235                 240                 245                 250
   acc acg atc cgt acg ctg aac cgc atg ggt tac cag acc gct gtt gtt    1179
   Thr Thr Ile Arg Thr Leu Asn Arg Met Gly Tyr Gln Thr Ala Val Val
                   255                 260                 265
   tcc ggt ggt ttc atc cag gtg ttg gaa ggt ttg gct gag gag ttg gag    1227
   Ser Gly Gly Phe Ile Gln Val Leu Glu Gly Leu Ala Glu Glu Leu Glu
               270                 275                 280
   ttg gat tat gtc cgc gcc aac act ttg gaa atc gtt gat ggc aag ctg    1275
   Leu Asp Tyr Val Arg Ala Asn Thr Leu Glu Ile Val Asp Gly Lys Leu
           285                 290                 295
   acc ggc aac gtc acc gga aag atc gtt gac cgc gct gcg aag gct gag    1323
   Thr Gly Asn Val Thr Gly Lys Ile Val Asp Arg Ala Ala Lys Ala Glu
       300                 305                 310
   ttc ctc cgt gag ttc gct gcg gat tct ggc ctg aag atg tac cag act    1371
   Phe Leu Arg Glu Phe Ala Ala Asp Ser Gly Leu Lys Met Tyr Gln Thr
   315                 320                 325                 330
   gtc gct gtc ggt gat ggc gct aat gac atc gat atg ctc tcc gct gcg    1419
   Val Ala Val Gly Asp Gly Ala Asn Asp Ile Asp Met Leu Ser Ala Ala
                   335                 340                 345
   ggt ctg ggt gtt gct ttc aac gcg aag cct gcg ctg aag gag att gcg    1467
   Gly Leu Gly Val Ala Phe Asn Ala Lys Pro Ala Leu Lys Glu Ile Ala
               350                 355                 360
   gat act tcc gtg aac cac cca ttc ctc gac gag gtt ttg cac atc atg    1515
   Asp Thr Ser Val Asn His Pro Phe Leu Asp Glu Val Leu His Ile Met
           365                 370                 375
   ggc att tcc cgc gac gag atc gat ctg gcg gat cag gaa gac ggc act    1563
   Gly Ile Ser Arg Asp Glu Ile Asp Leu Ala Asp Gln Glu Asp Gly Thr
       380                 385                 390
   ttc cac cgc gtt cca ttg acc aat gcc taaagattcg cttctcgacg          1610
   Phe His Arg Val Pro Leu Thr Asn Ala
   395                 400
   cccacctcct cctcaag                                                 1627
<210> 6
   <211> 403
   <212> PRT
   <213> C. glutanicum ATCC 13 032
<400> 6
   Met Ile Thr Val Ser Gly Lys Asp Arg Pro Gly Val Thr Ala Ala Phe
    1               5                  10                  15
   Phe Arg Val Leu Ser Ala Asn Gln Val Gln Val Leu Asp Val Glu Gln
               20                  25                  30
   Ser Met Phe Arg Gly Phe Leu Asn Leu Ala Ala Phe Val Gly Ile Ala
           35                  40                  45
   Pro Glu Arg Val Glu Thr Val Thr Thr Gly Leu Thr Asp Thr Leu Lys
       50                  55                  60
   Val His Gly Gln Ser Val Val Val Glu Leu Gln Glu Thr Val Gln Ser
   65                  70                  75                  80
   Ser Arg Pro Arg Ser Ser His Val Val Val Val Leu Gly Asp Pro Val
                   85                  90                  95
   Asp Ala Leu Asp Ile Ser Arg Ile Gly Gln Thr Leu Ala Asp Tyr Asp
               100                 105                 110
   Ala Asn Ile Asp Thr Ile Arg Gly Ile Ser Asp Tyr Pro Val Thr Gly
           115                 120                 125
   Leu Glu Leu Lys Val Thr Val Pro Asp Val Ser Pro Gly Gly Gly Glu
       130                 135                 140
   Ala Met Arg Lys Ala Leu Ala Ala Leu Thr Ser Glu Leu Asn Val Asp
   145                 150                 155                 160
   Ile Ala Ile Glu Arg Ser Gly Leu Leu Arg Arg Ser Lys Arg Leu Val
                   165                 170                 175
   Cys Phe Asp Cys Asp Ser Thr Leu Ile Thr Gly Glu Val Ile Glu Met
               180                 185                 190
   Leu Ala Ala His Ala Gly Lys Glu Ala Glu Val Ala Ala Val Thr Glu
           195                 200                 205
   Arg Ala Met Arg Gly Glu Leu Asp Phe Glu Glu Ser Leu Arg Glu Arg
       210                 215                 220
   Val Lys Ala Leu Ala Gly Leu Asp Ala Ser Val Ile Asp Glu Val Ala
   225                 230                 235                 240
   Ala Ala Ile Glu Leu Thr Pro Gly Ala Arg Thr Thr Ile Arg Thr Leu
                   245                 250                 255
   Asn Arg Met Gly Tyr Gln Thr Ala Val Val Ser Gly Gly Phe Ile Gln
               260                 265                 270
   Val Leu Glu Gly Leu Ala Glu Glu Leu Glu Leu Asp Tyr Val Arg Ala
           275                 280                 285
   Asn Thr Leu Glu Ile Val Asp Gly Lys Leu Thr Gly Asn Val Thr Gly
       290                 295                 300
   Lys Ile Val Asp Arg Ala Ala Lys Ala Glu Phe Leu Arg Glu Phe Ala
   305                 310                 315                 320
   Ala Asp Ser Gly Leu Lys Met Tyr Gln Thr Val Ala Val Gly Asp Gly
                   325                 330                 335
   Ala Asn Asp Ile Asp Met Leu Ser Ala Ala Gly Leu Gly Val Ala Phe
               340                 345                 350
   Asn Ala Lys Pro Ala Leu Lys Glu Ile Ala Asp Thr Ser Val Asn His
           355                 360                 365
   Pro Phe Leu Asp Glu Val Leu His Ile Met Gly Ile Ser Arg Asp Glu
       370                 375                 380
   Ile Asp Leu Ala Asp Gln Glu Asp Gly Thr Phe His Arg Val Pro Leu
   385                 390                 395                 400
   Thr Asn Ala
<210> 7
   <211> 1304
   <212> DNA
   <213> C. glutanicum ATCC 13 032
<220>
   <221> CDS
   <222> (131)..(1258)
   <223> ser C (Phosphoserin-phosphatase)
<400> 7
   catcgtttgg gagactgcgt gaagaatcta gggtgctagg aactgacagc ttcagggtta 60
   tagttgttgg gtcagatcgt taacgatccc tggccctttt acttccaagc gcagaaagtt 120
   gcccgaagac atg acc gac ttc ccc acc ctg ccc tct gag ttc atc cct    169
              Met Thr Asp Phe Pro Thr Leu Pro Ser Glu Phe Ile Pro
               1               5                  10
   ggc gac ggc cgt ttc ggc tgc gga cct tcc aag gtt cga cca gaa cag   217
   Gly Asp Gly Arg Phe Gly Cys Gly Pro Ser Lys Val Arg Pro Glu Gln
       15                  20                  25
   att cag gct att gtc gac gga tcc gca tcc gtc atc ggt acc tca cac   265
   Ile Gln Ala Ile Val Asp Gly Ser Ala Ser Val Ile Gly Thr Ser His
   30                  35                  40                  45
   cgt cag ccg gca gta aaa aac gtc gtg ggt tca atc cgc gag gga ctc   313
   Arg Gln Pro Ala Val Lys Asn Val Val Gly Ser Ile Arg Glu Gly Leu
                   50                  55                  60
   tcc gac ctc ttc tcc ctt cca gaa ggc tac gag atc atc ctt tcc cta   361
   Ser Asp Leu Phe Ser Leu Pro Glu Gly Tyr Glu Ile Ile Leu Ser Leu
               65                  70                  75
   ggt ggt gcg acc gca ttc tgg gat gca gca acc ttc gga ctc att gaa   409
   Gly Gly Ala Thr Ala Phe Trp Asp Ala Ala Thr Phe Gly Leu Ile Glu
           80                  85                  90
   aag aag tcc ggt cac ctt tct ttc ggt gag ttc tcc tcc aag ttc gca   457
   Lys Lys Ser Gly His Leu Ser Phe Gly Glu Phe Ser Ser Lys Phe Ala
       95                  100                 105
   aag gct tct aag ctt gct cct tgg ctc gac gag cca gag atc gtc acc   505
   Lys Ala Ser Lys Leu Ala Pro Trp Leu Asp Glu Pro Glu Ile Val Thr
   110                 115                 120                 125
   gca gaa acc ggt gac tct ccg gcc cca cag gca ttc gaa ggc gcc gat   553
   Ala Glu Thr Gly Asp Ser Pro Ala Pro Gln Ala Phe Glu Gly Ala Asp
                   130                 135                 140
   gtt att gca tgg gca cac aac gaa acc tcc gct ggc gcc atg gtt cca   601
   Val Ile Ala Trp Ala His Asn Glu Thr Ser Ala Gly Ala Met Val Pro
               145                 150                 155
   gtt ctt cgc ccc gaa ggc tct gaa ggc tcc ctg gtt gcc att gac gca   649
   Val Leu Arg Pro Glu Gly Ser Glu Gly Ser Leu Val Ala Ile Asp Ala
           160                 165                 170
   acc tcc ggc gct ggt gga ctg cca gta gac atc aag aac tcc gat gtt   697
   Thr Ser Gly Ala Gly Gly Leu Pro Val Asp Ile Lys Asn Ser Asp Val
       175                 180                 185
   tac tac ttc tcc cca cag aag tgc ttc gca tcc gac ggt ggc ctg tgg   745
   Tyr Tyr Phe Ser Pro Gln Lys Cys Phe Ala Ser Asp Gly Gly Leu Trp
   190                 195                 200                 205
   ctt gca gcg atg agc cca gca gct ctc gag cgc atc gag aag atc aac   793
   Leu Ala Ala Met Ser Pro Ala Ala Leu Glu Arg Ile Glu Lys Ile Asn
               210                   215                   220
   gct tcc gat cgc ttc atc cct gag ttc ctc aac ctg cag acc gca gtg     841
   Ala Ser Asp Arg Phe Ile Pro Glu Phe Leu Asn Leu Gln Thr Ala Val
               225                 230                 235
   gat aac tcc ctg aag aac cag acc tac aac acc cca gct gtt gct acc     889
   Asp Asn Ser Leu Lys Asn Gln Thr Tyr Asn Thr Pro Ala Val Ala Thr
           240                 245                 250
   ttg ctg atg ctg gac aac cag gtc aag tgg atg aac tcc aac ggc ggc     937
   Leu Leu Met Leu Asp Asn Gln Val Lys Trp Met Asn Ser Asn Gly Gly
       255                 260                 265
   ctg gat gga atg gtt gct cgc acc aca gca agc tcc tcc gcc ctg tac     985
   Leu Asp Gly Met Val Ala Arg Thr Thr Ala Ser Ser Ser Ala Leu Tyr
   270                 275                 280                 285
   aac tgg gct gag gct cgc gag gag gca tcc cca tac gtg gca gat gca     1033
   Asn Trp Ala Glu Ala Arg Glu Glu Ala Ser Pro Tyr Val Ala Asp Ala
                   290                 295                 300
   gct aag cgc tcc ctc gtt gtc ggc acc atc gac ttc gat gac tcc atc     1081
   Ala Lys Arg Ser Leu Val Val Gly Thr Ile Asp Phe Asp Asp Ser Ile
               305                 310                 315
   gac gca gca gtg atc gct aag ata ctg cgc gca aac ggc atc ctg gac     1129
   Asp Ala Ala Val Ile Ala Lys Ile Leu Arg Ala Asn Gly Ile Leu Asp
           320                 325                 330
   acc gag cct tac cgc aag ctg gga cgc aac cag ctg cgc atc ggt atg     1177
   Thr Glu Pro Tyr Arg Lys Leu Gly Arg Asn Gln Leu Arg Ile Gly Met
       335                 340                 345
   ttc cca gcg atc gat tcc acc gat gtg gaa aag ctc acc gga gca atc     1225
   Phe Pro Ala Ile Asp Ser Thr Asp Val Glu Lys Leu Thr Gly Ala Ile
   350                 355                 360                 365
   gac ttc atc ctc gat ggc ggt ttt gca agg aag taataccccc actttgaaaa 1278
   Asp Phe Ile Leu Asp Gly Gly Phe Ala Arg Lys
                   370                 375
   acaccccgta cagttacacc agtacg                                        1304
<210> 8
   <211> 376
   <212> PRT
   <213> C. glutanicum ATCC 13 032
<400> 8
   Met Thr Asp Phe Pro Thr Leu Pro Ser Glu Phe Ile Pro Gly Asp Gly
    1               5                  10                  15
   Arg Phe Gly Cys Gly Pro Ser Lys Val Arg Pro Glu Gln Ile Gln Ala
               20                  25                  30
   Ile Val Asp Gly Ser Ala Ser Val Ile Gly Thr Ser His Arg Gln Pro
           35                  40                  45
   Ala Val Lys Asn Val Val Gly Ser Ile Arg Glu Gly Leu Ser Asp Leu
   50 55 60
   Phe Ser Leu Pro Glu Gly Tyr Glu Ile Ile Leu Ser Leu Gly Gly Ala
   65 70 75 80
   Thr Ala Phe Trp Asp Ala Ala Thr Phe Gly Leu Ile Glu Lys Lys Ser
   85 90 95
   Gly His Leu Ser Phe Gly Glu Phe Ser Ser Lys Phe Ala Lys Ala Ser
   100 105 110
   Lys Leu Ala Pro Trp Leu Asp Glu Pro Glu Ile Val Thr Ala Glu Thr
   115 120 125
   Gly Asp Ser Pro Ala Pro Gln Ala Phe Glu Gly Ala Asp Val Ile Ala
   130 135 140
   Trp Ala His Asn Glu Thr Ser Ala Gly Ala Met Val Pro Val Leu Arg
   145 150 155 160
   Pro Glu Gly Ser Glu Gly Ser Leu Val Ala Ile Asp Ala Thr Ser Gly
   165 170 175
   Ala Gly Gly Leu Pro Val Asp Ile Lys Asn Ser Asp Val Tyr Tyr Phe
   180 185 190
   Ser Pro Gln Lys Cys Phe Ala Ser Asp Gly Gly Leu Trp Leu Ala Ala
   195 200 205
   Met Ser Pro Ala Ala Leu Glu Arg Ile Glu Lys Ile Asn Ala Ser Asp
   210 215 220
   Arg Phe Ile Pro Glu Phe Leu Asn Leu Gln Thr Ala Val Asp Asn Ser
   225 230 235 240
   Leu Lys Asn Gln Thr Tyr Asn Thr Pro Ala Val Ala Thr Leu Leu Met
   245 250 255
   Leu Asp Asn Gln Val Lys Trp Met Asn Ser Asn Gly Gly Leu Asp Gly
   260 265 270
   Met Val Ala Arg Thr Thr Ala Ser Ser Ser Ala Leu Tyr Asn Trp Ala
   275 280 285
   Glu Ala Arg Glu Glu Ala Ser Pro Tyr Val Ala Asp Ala Ala Lys Arg
   290 295 300
   Ser Leu Val Val Gly Thr Ile Asp Phe Asp Asp Ser Ile Asp Ala Ala
   305 310 315 320
   Val Ile Ala Lys Ile Leu Arg Ala Asn Gly Ile Leu Asp Thr Glu Pro
   325 330 335
   Tyr Arg Lys Leu Gly Arg Asn Gln Leu Arg Ile Gly Met Phe Pro Ala
   340 345 350
   Ile Asp Ser Thr Asp Val Glu Lys Leu Thr Gly Ala Ile Asp Phe Ile
   355 360 365
   Leu Asp Gly Gly Phe Ala Arg Lys
   370 375
<210> 9
   <211> 2817
   <212> DNA
   <213> C. glutamicum ATCC 14 752
<220>
   <221> CDS
   <222> (398)..(1867)
   <223> thr E (Threonin-exportcarrier)
<400> 9
   aatgaaataa tcccctcacc aactggcgac attcaaacac cgtttcattt ccaaacatcg 60
   agccaaggga aaagaaagcc cctaagcccc gtgttattaa atggagactc tttggagacc 120
   tcaagccaaa aaggggcatt ttcattaaga aaatacccct ttgacctggt gttattgagc 180
   tggagaagag acttgaactc tcaacctacg cattacaagt gcgttgcgct gccaattgcg 240
   ccactccagc accgcagatg ctgatgatca acaactacga atacgtatct tagcgtatgt 300
   gtacatcaca atggaattcg gggctagagt atctggtgaa ccgtgcataa acgacctgtg 360
   attggactct ttttccttgc aaaatgtttt ccagcgg atg ttg agt ttt gcg acc  415
                                           Met Leu Ser Phe Ala Thr
                                            1               5
   ctt cgt ggc cgc att tca aca gtt gac gct gca aaa gcc gca cct ccg   463
   Leu Arg Gly Arg Ile Ser Thr Val Asp Ala Ala Lys Ala Ala Pro Pro
               10                  15                  20
   cca tcg cca cta gcc ccg att gat ctc act gac cat agt caa gtg gcc   511
   Pro Ser Pro Leu Ala Pro Ile Asp Leu Thr Asp His Ser Gln Val Ala
           25                  30                  35
   ggt gtg atg aat ttg gct gcg aga att ggc gat att ttg ctt tct tca   559
   Gly Val Met Asn Leu Ala Ala Arg Ile Gly Asp Ile Leu Leu Ser Ser
       40                  45                  50
   ggt acg tca aac agt gat acc aag gtg caa gtt cga gcg gtg acc tct   607
   Gly Thr Ser Asn Ser Asp Thr Lys Val Gln Val Arg Ala Val Thr Ser
   55                  60                  65                  70
   gcg tat ggc ctg tac tat acg cat gtg gat atc acg ttg aat acg atc   655
   Ala Tyr Gly Leu Tyr Tyr Thr His Val Asp Ile Thr Leu Asn Thr Ile
                   75                  80                  85
   acc atc ttc acc aac atc ggt gtg gag agg aag atg ccg gtc aac gtg   703
   Thr Ile Phe Thr Asn Ile Gly Val Glu Arg Lys Met Pro Val Asn Val
               90                  95                  100
   ttt cat gtt gtg ggc aag ttg gac acc aac ttc tcc aaa ctg tct gag   751
   Phe His Val Val Gly Lys Leu Asp Thr Asn Phe Ser Lys Leu Ser Glu
           105                 110                 115
   gtt gac cgt ttg atc cgt tcc att cag gct ggt gct acc ccg cct gag     799
   Val Asp Arg Leu Ile Arg Ser Ile Gln Ala Gly Ala Thr Pro Pro Glu
       120                 125                 130
   gtt gcc gag aaa att ctg gac gag ttg gag caa tcg cct gcg tct tat     847
   Val Ala Glu Lys Ile Leu Asp Glu Leu Glu Gln Ser Pro Ala Ser Tyr
   135                 140                 145                 150
   ggt ttc cct gtt gcg ttg ctt ggc tgg gca atg atg ggt ggc gct gtt     895
   Gly Phe Pro Val Ala Leu Leu Gly Trp Ala Met Met Gly Gly Ala Val
                   155                 160                 165
   gct gtg ctg ttg ggt ggt gga tgg cag gtt tcc cta att gct ttt att     943
   Ala Val Leu Leu Gly Gly Gly Trp Gln Val Ser Leu Ile Ala Phe Ile
               170                 175                 180
   acc gcg ttc acg atc att gcc acg acg tca ttt ttg gga aag aag ggt     991
   Thr Ala Phe Thr Ile Ile Ala Thr Thr Ser Phe Leu Gly Lys Lys Gly
           185                 190                 195
   ttg cct act ttc ttc caa aat gtt gtt ggt ggt ttt att gcc acg ctg    1039
   Leu Pro Thr Phe Phe Gln Asn Val Val Gly Gly Phe Ile Ala Thr Leu
       200                 205                 210
   cct gca tcg att gct tat tct ttg gcg ttg caa ttt ggt ctt gag atc    1087
   Pro Ala Ser Ile Ala Tyr Ser Leu Ala Leu Gln Phe Gly Leu Glu Ile
   215                 220                 225                 230
   aaa ccg agc cag atc atc gca tct gga att gtt gtg ctg ttg gca ggt    1135
   Lys Pro Ser Gln Ile Ile Ala Ser Gly Ile Val Val Leu Leu Ala Gly
                   235                 240                 245
   ttg aca ctt gtg caa tct ctg cag gac ggc atc acg ggc gct ccg gtg    1183
   Leu Thr Leu Val Gln Ser Leu Gln Asp Gly Ile Thr Gly Ala Pro Val
               250                 255                 260
   aca gca agt gca cga ttt ttt gaa aca ctc ctg ttt acc ggc ggc att    1231
   Thr Ala Ser Ala Arg Phe Phe Glu Thr Leu Leu Phe Thr Gly Gly Ile
           265                 270                 275
   gtt gct ggc gtg ggt ttg ggc att cag ctt tct gaa atc ttg cat gtc    1279
   Val Ala Gly Val Gly Leu Gly Ile Gln Leu Ser Glu Ile Leu His Val
       280                 285                 290
   atg ttg cct gcc atg gag tcc gct gca gca cct aat tat tcg tct aca    1327
   Met Leu Pro Ala Met Glu Ser Ala Ala Ala Pro Asn Tyr Ser Ser Thr
   295                 300                 305                 310
   ttc gcc cgc att atc gct ggt ggc gtc acc gca gcg gcc ttc gca gtg    1375
   Phe Ala Arg Ile Ile Ala Gly Gly Val Thr Ala Ala Ala Phe Ala Val
                   315                 320                 325
   ggt tgt tac gcg gag tgg tcc tcg gtg att att gcg ggg ctt act gcg    1423
   Gly Cys Tyr Ala Glu Trp Ser Ser Val Ile Ile Ala Gly Leu Thr Ala
               330                 335                 340
   ctg atg ggt tct gcg ttt tat tac ctc ttc gtt gtt tat tta ggc ccc    1471
   Leu Met Gly Ser Ala Phe Tyr Tyr Leu Phe Val Val Tyr Leu Gly Pro
           345                 350                 355
   gtc tct gcc gct gcg att gct gca aca gca gtt ggt ttc act ggt ggt    1519
   Val Ser Ala Ala Ala Ile Ala Ala Thr Ala Val Gly Phe Thr Gly Gly
       360                 365                 370
   ttg ctt gcc cgt cga ttc ttg att cca ccg ttg att gtg gcg att gcc    1567
   Leu Leu Ala Arg Arg Phe Leu Ile Pro Pro Leu Ile Val Ala Ile Ala
   375                 380                 385                 390
   ggc atc aca cca atg ctt cca ggt cta gca att tac cgc gga atg tac    1615
   Gly Ile Thr Pro Met Leu Pro Gly Leu Ala Ile Tyr Arg Gly Met Tyr
                   395                 400                 405
   gcc acc ttg aat gat caa aca ctc atg ggt ttc acc aac att gcg gtt    1663
   Ala Thr Leu Asn Asp Gln Thr Leu Met Gly Phe Thr Asn Ile Ala Val
               410                 415                 420
   gct tta gcc act gct tca tca ctt gcc gct ggc gtg gtt ttg ggt gag    1711
   Ala Leu Ala Thr Ala Ser Ser Leu Ala Ala Gly Val Val Leu Gly Glu
           425                 430                 435
   tgg att gcc cgc agg cta cgt cgt cca cca cgc ttc aac cca tac cgt    1759
   Trp Ile Ala Arg Arg Leu Arg Arg Pro Pro Arg Phe Asn Pro Tyr Arg
       440                 445                 450
   gca ttt acc aag gcg aat gag ttc tcc ttc cag gag gaa gct gag cag    1807
   Ala Phe Thr Lys Ala Asn Glu Phe Ser Phe Gln Glu Glu Ala Glu Gln
   455                 460                 465                 470
   aat cag cgc cgg cag aga aaa cgt cca aag act aat caa aga ttc ggt    1855
   Asn Gln Arg Arg Gln Arg Lys Arg Pro Lys Thr Asn Gln Arg Phe Gly
                   475                 480                 485
   aat aaa agg taa aaatcaacct gcttaggcgt ctttcgctta aatagcgtag        1907
   Asn Lys Arg
               490
   aatatcgggt cgatcgcttt taaacactca ggaggatcct tgccggccaa aatcacggac 1967
   actcgtccca ccccagaatc ccttcacgct gttgaagagg aaaccgcagc cggtgcccgc 2027
   aggattgttg ccacctattc taaggacttc ttcgacggcg tcactttgat gtgcatgctc 2087
   ggcgttgaac ctcagggcct gcgttacacc aaggtcgctt ctgaacacga ggaagctcag 2147
   ccaaagaagg ctacaaagcg gactcgtaag gcaccagcta agaaggctgc tgctaagaaa 2207
   acgaccaaga agaccactaa gaaaactact aaaaagacca ccgcaaagaa gaccacaaag 2267
   aagtcttaag ccggatctta tatggatgat tccaatagct ttgtagttgt tgctaaccgt 2327
   ctgccagtgg atatgactgt ccacccagat ggtagctata gcatctcccc cagccccggt 2387
   ggccttgtca cggggctttc ccccgttctg gaacaacatc gtggatgttg ggtcggatgg 2447
   cctggaactg tagatgttgc acccgaacca tttcgaacag atacgggtgt tttgctgcac 2507
   cctgttgtcc tcactgcaag tgactatgaa ggcttctacg agggcttttc aaacgcaacg 2567
   ctgtggcctc ttttccacga tttgattgtt actccggtgt acaacaccga ttggtggcat 2627
   gcgtttcggg aagtaaacct caagttcgct gaagccgtga gccaagtggc ggcacacggt 2687
   gccactgtgt gggtgcagga ctatcagctg ttgctggttc ctggcatttt gcgccagatg 2747
   cgccctgatt tgaagatcgg tttcttcctc cacattccct tcccttcccc tgatctgttc 2807
   cgtcagctgc 2817
<210> 10
   <211> 489
   <212> PRT
   <213> C. glutamicum ATCC 14 752
<400> 10
   Met Leu Ser Phe Ala Thr Leu Arg Gly Arg Ile Ser Thr Val Asp Ala
    1               5                  10                  15
   Ala Lys Ala Ala Pro Pro Pro Ser Pro Leu Ala Pro Ile Asp Leu Thr
               20                  25                  30
   Asp His Ser Gln Val Ala Gly Val Met Asn Leu Ala Ala Arg Ile Gly
           35                  40                  45
   Asp Ile Leu Leu Ser Ser Gly Thr Ser Asn Ser Asp Thr Lys Val Gln
       50                  55                  60
   Val Arg Ala Val Thr Ser Ala Tyr Gly Leu Tyr Tyr Thr His Val Asp
   65                  70                  75                  80
   Ile Thr Leu Asn Thr Ile Thr Ile Phe Thr Asn Ile Gly Val Glu Arg
                   85                  90                  95
   Lys Met Pro Val Asn Val Phe His Val Val Gly Lys Leu Asp Thr Asn
               100                 105                 110
   Phe Ser Lys Leu Ser Glu Val Asp Arg Leu Ile Arg Ser Ile Gln Ala
           115                 120                 125
   Gly Ala Thr Pro Pro Glu Val Ala Glu Lys Ile Leu Asp Glu Leu Glu
       130                 135                 140
   Gln Ser Pro Ala Ser Tyr Gly Phe Pro Val Ala Leu Leu Gly Trp Ala
   145                 150                 155                 160
   Met Met Gly Gly Ala Val Ala Val Leu Leu Gly Gly Gly Trp Gln Val
                   165                 170                 175
   Ser Leu Ile Ala Phe Ile Thr Ala Phe Thr Ile Ile Ala Thr Thr Ser
               180                 185                 190
   Phe Leu Gly Lys Lys Gly Leu Pro Thr Phe Phe Gln Asn Val Val Gly
           195                 200                 205
   Gly Phe Ile Ala Thr Leu Pro Ala Ser Ile Ala Tyr Ser Leu Ala Leu
       210                 215                 220
   Gln Phe Gly Leu Glu Ile Lys Pro Ser Gln Ile Ile Ala Ser Gly Ile
   225                 230                 235                 240
   Val Val Leu Leu Ala Gly Leu Thr Leu Val Gln Ser Leu Gln Asp Gly
                   245                 250                 255
   Ile Thr Gly Ala Pro Val Thr Ala Ser Ala Arg Phe Phe Glu Thr Leu
               260                 265                 270
   Leu Phe Thr Gly Gly Ile Val Ala Gly Val Gly Leu Gly Ile Gln Leu
           275                 280                 285
   Ser Glu Ile Leu His Val Met Leu Pro Ala Met Glu Ser Ala Ala Ala
       290                 295                 300
   Pro Asn Tyr Ser Ser Thr Phe Ala Arg Ile Ile Ala Gly Gly Val Thr
   305                 310                 315                 320
   Ala Ala Ala Phe Ala Val Gly Cys Tyr Ala Glu Trp Ser Ser Val Ile
                   325                 330                 335
   Ile Ala Gly Leu Thr Ala Leu Met Gly Ser Ala Phe Tyr Tyr Leu Phe
               340                 345                 350
   Val Val Tyr Leu Gly Pro Val Ser Ala Ala Ala Ile Ala Ala Thr Ala
           355                 360                 365
   Val Gly Phe Thr Gly Gly Leu Leu Ala Arg Arg Phe Leu Ile Pro Pro
       370                 375                 380
   Leu Ile Val Ala Ile Ala Gly Ile Thr Pro Met Leu Pro Gly Leu Ala
   385                 390                 395                 400
   Ile Tyr Arg Gly Met Tyr Ala Thr Leu Asn Asp Gln Thr Leu Met Gly
                   405                 410                 415
   Phe Thr Asn Ile Ala Val Ala Leu Ala Thr Ala Ser Ser Leu Ala Ala
               420                 425                 430
   Gly Val Val Leu Gly Glu Trp Ile Ala Arg Arg Leu Arg Arg Pro Pro
           435                 440                 445
   Arg Phe Asn Pro Tyr Arg Ala Phe Thr Lys Ala Asn Glu Phe Ser Phe
       450                 455                 460
   Gln Glu Glu Ala Glu Gln Asn Gln Arg Arg Gln Arg Lys Arg Pro Lys
   465                 470                 475                 480
   Thr Asn Gln Arg Phe Gly Asn Lys Arg
                   485
<210> 11
   <211> 1909
   <212> DNA
   <213> C. glutanicum ATCC 13 032
<220>
   <221> CDS
   <222> (280)..(1746)
   <223> thr E (Threonin-exportcarrier)
<400> 11
   agcttgcatg cctgcaggtc gactctagag gatccccccc ctttgacctg gtgttattga 60
   gctggagaag agacttgaac tctcaaccta cgcattacaa gtgcgttgcg ctgccaattg 120
   cgccactcca gcaccgcaga tgctgatgat caacaactac gaatacgtat cttagcgtat 180
   gtgtacatca caatggaatt cggggctaga gtatctggtg aaccgtgcat aaacgacctg 240
   tgattggact ctttttcctt gcaaaatgtt ttccagcgg atg ttg agt ttt gcg    294
                                              Met Leu Ser Phe Ala
                                              1               5
   acc ctt cgt ggc cgc att tca aca gtt gac gct gca aaa gcc gca cct   342
   Thr Leu Arg Gly Arg Ile Ser Thr Val Asp Ala Ala Lys Ala Ala Pro
                   10                  15                  20
   ccg cca tcg cca cta gcc ccg att gat ctc act gac cat agt caa gtg   390
   Pro Pro Ser Pro Leu Ala Pro Ile Asp Leu Thr Asp His Ser Gln Val
               25                  30                  35
   gcc ggt gtg atg aat ttg gct gcg aga att ggc gat att ttg ctt tct   438
   Ala Gly Val Met Asn Leu Ala Ala Arg Ile Gly Asp Ile Leu Leu Ser
           40                  45                  50
   tca ggt acg tca aat agt gac acc aag gta caa gtt cga gca gtg acc   486
   Ser Gly Thr Ser Asn Ser Asp Thr Lys Val Gln Val Arg Ala Val Thr
       55                  60                  65
   tct gcg tac ggt ttg tac tac acg cac gtg gat atc acg ttg aat acg   534
   Ser Ala Tyr Gly Leu Tyr Tyr Thr His Val Asp Ile Thr Leu Asn Thr
    70                 75                  80                  85
   atc acc atc ttc acc aac atc ggt gtg gag agg aag atg ccg gtc aac    582
   Ile Thr Ile Phe Thr Asn Ile Gly Val Glu Arg Lys Met Pro Val Asn
                   90                  95                  100
   gtg ttt cat gtt gta ggc aag ttg gac acc aac ttc tcc aaa ctg tct    630
   Val Phe His Val Val Gly Lys Leu Asp Thr Asn Phe Ser Lys Leu Ser
               105                 110                 115
   gag gtt gac cgt ttg atc cgt tcc att cag gct ggt gcg acc ccg cct    678
   Glu Val Asp Arg Leu Ile Arg Ser Ile Gln Ala Gly Ala Thr Pro Pro
           120                 125                 130
   gag gtt gcc gag aaa atc ctg gac gag ttg gag caa tcc cct gcg tct    726
   Glu Val Ala Glu Lys Ile Leu Asp Glu Leu Glu Gln Ser Pro Ala Ser
       135                 140                 145
   tat ggt ttc cct gtt gcg ttg ctt ggc tgg gca atg atg ggt ggt gct    774
   Tyr Gly Phe Pro Val Ala Leu Leu Gly Trp Ala Met Met Gly Gly Ala
   150                 155                 160                 165
   gtt gct gtg ctg ttg ggt ggt gga tgg cag gtt tcc cta att gct ttt    822
   Val Ala Val Leu Leu Gly Gly Gly Trp Gln Val Ser Leu Ile Ala Phe
                   170                 175                 180
   att acc gcg ttc acg atc att gcc acg acg tca ttt ttg gga aag aag    870
   Ile Thr Ala Phe Thr Ile Ile Ala Thr Thr Ser Phe Leu Gly Lys Lys
               185                 190                 195
   ggt ttg cct act ttc ttc caa aat gtt gtt ggt ggt ttt att gcc acg    918
   Gly Leu Pro Thr Phe Phe Gln Asn Val Val Gly Gly Phe Ile Ala Thr
           200                 205                 210
   ctg cct gca tcg att gct tat tct ttg gcg ttg caa ttt ggt ctt gag    966
   Leu Pro Ala Ser Ile Ala Tyr Ser Leu Ala Leu Gln Phe Gly Leu Glu
       215                 220                 225
   atc aaa ccg agc cag atc atc gca tct gga att gtt gtg ctg ttg gca    1014
   Ile Lys Pro Ser Gln Ile Ile Ala Ser Gly Ile Val Val Leu Leu Ala
   230                 235                 240                 245
   ggt ttg aca ctc gtg caa tct ctg cag gac ggc atc acg ggc gct ccg    1062
   Gly Leu Thr Leu Val Gln Ser Leu Gln Asp Gly Ile Thr Gly Ala Pro
                   250                 255                 260
   gtg aca gca agt gca cga ttt ttc gaa aca ctc ctg ttt acc ggc ggc    1110
   Val Thr Ala Ser Ala Arg Phe Phe Glu Thr Leu Leu Phe Thr Gly Gly
               265                 270                 275
   att gtt gct ggc gtg ggt ttg ggc att cag ctt tct gaa atc ttg cat    1158
   Ile Val Ala Gly Val Gly Leu Gly Ile Gln Leu Ser Glu Ile Leu His
           280                 285                 290
   gtc atg ttg cct gcc atg gag tcc gct gca gca cct aat tat tcg tct    1206
   Val Met Leu Pro Ala Met Glu Ser Ala Ala Ala Pro Asn Tyr Ser Ser
       295                 300                 305
   aca ttc gcc cgc att atc gct ggt ggc gtc acc gca gcg gcc ttc gca    1254
   Thr Phe Ala Arg Ile Ile Ala Gly Gly Val Thr Ala Ala Ala Phe Ala
   310                 315                 320                 325
   gtg ggt tgt tac gcg gag tgg tcc tcg gtg att att gcg ggg ctt act    1302
   Val Gly Cys Tyr Ala Glu Trp Ser Ser Val Ile Ile Ala Gly Leu Thr
                   330                 335                 340
   gcg ctg atg ggt tct gcg ttt tat tac ctc ttc gtt gtt tat tta ggc    1350
   Ala Leu Met Gly Ser Ala Phe Tyr Tyr Leu Phe Val Val Tyr Leu Gly
               345                 350                 355
   ccc gtc tct gcc gct gcg att gct gca aca gca gtt ggt ttc act ggt    1398
   Pro Val Ser Ala Ala Ala Ile Ala Ala Thr Ala Val Gly Phe Thr Gly
           360                 365                 370
   ggt ttg ctt gcc cgt cga ttc ttg att cca ccg ttg att gtg gcg att    1446
   Gly Leu Leu Ala Arg Arg Phe Leu Ile Pro Pro Leu Ile Val Ala Ile
       375                 380                 385
   gcc ggc atc aca cca atg ctt cca ggt cta gca att tac cgc gga atg    1494
   Ala Gly Ile Thr Pro Met Leu Pro Gly Leu Ala Ile Tyr Arg Gly Met
   390                 395                 400                 405
   tac gcc acc ctg aat gat caa aca ctc atg ggt ttc acc aac att gcg    1542
   Tyr Ala Thr Leu Asn Asp Gln Thr Leu Met Gly Phe Thr Asn Ile Ala
                   410                 415                 420
   gtt gct tta gcc act gct tca tca ctt gcc gct ggc gtg gtt ttg ggt    1590
   Val Ala Leu Ala Thr Ala Ser Ser Leu Ala Ala Gly Val Val Leu Gly
               425                 430                 435
   gag tgg att gcc cgc agg cta cgt cgt cca cca cgc ttc aac cca tac    1638
   Glu Trp Ile Ala Arg Arg Leu Arg Arg Pro Pro Arg Phe Asn Pro Tyr
           440                 445                 450
   cgt gca ttt acc aag gcg aat gag ttc tcc ttc cag gag gaa gct gag    1686
   Arg Ala Phe Thr Lys Ala Asn Glu Phe Ser Phe Gln Glu Glu Ala Glu
       455                 460                 465
   cag aat cag cgc cgg cag aga aaa cgt cca aag act aat cag aga ttc    1734
   Gln Asn Gln Arg Arg Gln Arg Lys Arg Pro Lys Thr Asn Gln Arg Phe
   470                 475                 480                 485
   ggt aat aaa agg taaaaatcaa cctgcttagg cgtctttcgc ttaaatagcg        1786
   Gly Asn Lys Arg
   tagaatatcg ggtcgatcgc ttttaaacac tcaggaggat ccttgccggc caaaatcacg 1846
   gacactcgtc ccaccccaga atcccttcac gctgttgaag aggaaaccgc agccggggta 1906
   ccg                                                                1909
<210> 12
   <211> 489
   <212> PRT
   <213> C. glutanicum ATCC 13 032
<400> 12
   Met Leu Ser Phe Ala Thr Leu Arg Gly Arg Ile Ser Thr Val Asp Ala
   1 5 10 15
   Ala Lys Ala Ala Pro Pro Pro Ser Pro Leu Ala Pro Ile Asp Leu Thr
   20 25 30
   Asp His Ser Gln Val Ala Gly Val Met Asn Leu Ala Ala Arg Ile Gly
   35 40 45
   Asp Ile Leu Leu Ser Ser Gly Thr Ser Asn Ser Asp Thr Lys Val Gln
   50 55 60
   Val Arg Ala Val Thr Ser Ala Tyr Gly Leu Tyr Tyr Thr His Val Asp
   65 70 75 80
   Ile Thr Leu Asn Thr Ile Thr Ile Phe Thr Asn Ile Gly Val Glu Arg
   85 90 95
   Lys Met Pro Val Asn Val Phe His Val Val Gly Lys Leu Asp Thr Asn
   100 105 110
   Phe Ser Lys Leu Ser Glu Val Asp Arg Leu Ile Arg Ser Ile Gln Ala
   115 120 125
   Gly Ala Thr Pro Pro Glu Val Ala Glu Lys Ile Leu Asp Glu Leu Glu
   130 135 140
   Gln Ser Pro Ala Ser Tyr Gly Phe Pro Val Ala Leu Leu Gly Trp Ala
   145 150 155 160
   Met Met Gly Gly Ala Val Ala Val Leu Leu Gly Gly Gly Trp Gln Val
   165 170 175
   Ser Leu Ile Ala Phe Ile Thr Ala Phe Thr Ile Ile Ala Thr Thr Ser
   180 185 190
   Phe Leu Gly Lys Lys Gly Leu Pro Thr Phe Phe Gln Asn Val Val Gly
   195 200 205
   Gly Phe Ile Ala Thr Leu Pro Ala Ser Ile Ala Tyr Ser Leu Ala Leu
   210 215 220
   Gln Phe Gly Leu Glu Ile Lys Pro Ser Gln Ile Ile Ala Ser Gly Ile
   225 230 235 240
   Val Val Leu Leu Ala Gly Leu Thr Leu Val Gln Ser Leu Gln Asp Gly
   245 250 255
   Ile Thr Gly Ala Pro Val Thr Ala Ser Ala Arg Phe Phe Glu Thr Leu
   260 265 270
   Leu Phe Thr Gly Gly Ile Val Ala Gly Val Gly Leu Gly Ile Gln Leu
   275 280 285
   Ser Glu Ile Leu His Val Met Leu Pro Ala Met Glu Ser Ala Ala Ala
   290 295 300
   Pro Asn Tyr Ser Ser Thr Phe Ala Arg Ile Ile Ala Gly Gly Val Thr
   305 310 315 320
   Ala Ala Ala Phe Ala Val Gly Cys Tyr Ala Glu Trp Ser Ser Val Ile
                   325                     330                     335
   Ile Ala Gly Leu Thr Ala Leu Met Gly Ser Ala Phe Tyr Tyr Leu Phe
               340                     345                     350
   Val Val Tyr Leu Gly Pro Val Ser Ala Ala Ala Ile Ala Ala Thr Ala
           355                     360                     365
   Val Gly Phe Thr Gly Gly Leu Leu Ala Arg Arg Phe Leu Ile Pro Pro
       370                     375                     380
   Leu Ile Val Ala Ile Ala Gly Ile Thr Pro Met Leu Pro Gly Leu Ala
   385                     390                     395                     400
   Ile Tyr Arg Gly Met Tyr Ala Thr Leu Asn Asp Gln Thr Leu Met Gly
                   405                     410                     415
   Phe Thr Asn Ile Ala Val Ala Leu Ala Thr Ala Ser Ser Leu Ala Ala
               420                     425                     430
   Gly Val Val Leu Gly Glu Trp Ile Ala Arg Arg Leu Arg Arg Pro Pro
           435                     440                     445
   Arg Phe Asn Pro Tyr Arg Ala Phe Thr Lys Ala Asn Glu Phe Ser Phe
       450                     455                     460
   Gln Glu Glu Ala Glu Gln Asn Gln Arg Arg Gln Arg Lys Arg Pro Lys
   465                     470                     475                     480
   Thr Asn Gln Arg Phe Gly Asn Lys Arg
                   485

## Beschreibung

Die vorliegende Erfindung betrifft ein Verfahren zur mikrobiellen Herstellung von L-Serin und einen dazu geeigneten genetisch veränderten Mikroorganismus. Ferner umfaßt die vorliegende Erfindung die Verwendung von L-Serin und/oder deren Folgeprodukte in der Nahrungs-, Futtermittel- und/oder Pharmaindustrie und/oder in der Humanmedizin.

Aminosäuren, wie beispielsweise L-Glutamat, L-Lysin oder auch verzweigtkettige L-Aminosäuren sind in den letzten Jahren zunehmend in den Focus wirtschaftlichen Interesses gerückt. Dies gilt gleichermaßen auch für die Aminosäure L-Serin, die nicht nur als Vorstufe zur Synthese der aliphatischen Aminosäuren L-Glycin oder L-Cystein, sondern auch zur Herstellung von L-Tryptophan aus Indol und L-Serin dient. Hierbei wird für die Aminosäure L-Serin insbesondere in der Nahrungsmittel- Futtermittel- und Pharmaindustrie sowie in weiten Bereichen der Humanmedizin ein zunehmend wirtschaftliches Potential gesehen.

Verfahren zur mikrobiellen Herstellung von L-Serin sind in der Literatur zahlreich beschrieben. Auch sind bereits Fermentationen coryneformer Bakterien zur Herstellung von L-Serin bekannt. So ist beispielsweise ein Stamm von *Corynebacterium glycinophilum* in der Lage L-Serin aus Glycin und Kohlenhydraten zu bilden (Kubota K, Kageyama K, Shiro T und Okumura S (1971) Journal of General Applications in Microbiology, 17: 167-168; Kubota K, Kageyama K, Maeyashiki I, Yamada K und Okumura S (1972) Journal of General Applications in Microbiology 18: 365). An der Umsetzung von Glycin zu L-Serin ist hier das Enzym L-Serin-Hydroxymethyltransferase beteiligt (Kubota K und Yokozeki K (1989) Journal of Fermentation and Bioengeneering, 67(6):387-390). Die verwendeten Bakterienstämme weisen ferner einen verminderten Serin-Abbau auf, der auf eine Verringerung der Aktivität des Enzyms L-Serin-Dehydratase zurückzuführen ist (Kubota K, Kageyama K, Shiro T und Okumura S (1971) Journal of General Applications in Microbiology, 17: 167-168; Kubota K (1985) Agricultural Biological Chemistry, 49:7-12).

Weiterhin ist die fermentative Herstellung von L-Serin aus Methanol und Glycin mit Hilfe methylotropher Bakterien, beispielsweise der Gattung *Hyphomicrobium* bei lzumi Y, Yoshida T, Miyazaki SS, Mitsunaga T, Ohshiro T, Shiamo M, Miyata A und Tanabe T (1993) Applied Microbiology and Biotechnology, 39: 427-432 beschrieben.

In den zuvor genannten Fällen ist allerdings der Zusatz der Aminosäure Glycin als Vorstufe für die Bildung der Aminosäure L-Serin ausgehend von Kohlenhydraten erforderlich.

Demgegenüber sind bereits Verfahren zur Fermentation coryneformer Bakterien bekannt, die L-Serin direkt aus Kohlenhydraten, ohne zusätzliche Beigabe weiterer Vorstufen produzieren können. So sind z. B. Bakterienstämme der Gattung *Corynebacterium* und insbesondere der Art *Corynebacterium glutamicum* bei Yoshida H und Nakayama K (1974) Nihon-Nogei-Kagakukaishi 48: 201-208 beschrieben, die durch eine ungerichtete Mutagenese erhalten wurden und sich u. a. dadurch auszeichnen, daß sie resistent gegenüber den L-Serin-Analoga Serin-Hydroxamat bzw. β-Chloroalanin sind. Dies führt u. a. dazu, daß der Stoffwechselfluß vermehrt in Richtung L-Serin Biosynthese fließen kann, da die Aktivität der entsprechenden Enzyme einer geringeren Endprodukthemmung unterliegen.

Aus der EP 0 931 833 sind ferner Bakterienstämme der Spezies *Brevibacterium flavum* bekannt, die ebenfalls durch ungerichtete Mutagenese erhalten wurden und aufgrund dessen Defekte im Serin-Abbau aufweisen. Ferner weisen die dort beschriebenen Stämme ein verändertes serA-Gen auf, welches für eine feedback-desensitive 3-Phosphoglycerat-Dehydrogenase kodiert. Zusätzlich enthalten diese Stämme die aus dem heterologen Organismus *Escherichia coli* stammenden Gene *serB* und *serC*, die für die Enzyme Phosphoserin-Phosphatase bzw. Phosphoserin-Aminotransferase kodieren. Das hier beschriebene System weist somit eine hohe Komplexität hinsichtlich der Vielzahl zusätzlich eingeführter, z. T. heterologer Genstrukturen auf, verbunden mit einer genetischen Unbestimmtheit der Bakterienstämme hinsichtlich der eingangs erwähnten ungerichteten Mutagenese. Dies birgt die Gefahr einer relativ hohen Instabilität solcher Bakterienstämme im Verlauf eines Produktionsverfahrens im großtechnischen Maßstab in sich. Ferner ist beschrieben, daß durch die hier dargestellten Bakterienstämme lediglich eine um den Faktor 2 bis maximal Faktor 5 gesteigerte L-Serin-Produktion erreicht wird. Dies kann u. a. in einer suboptimalen Expression heterologer Gene begründet sein.
Ein weiterer Nachteil heterologer Systeme liegt in der geringen Akzeptanz von Fremd-DNA enthaltenden Systemen, insbesondere zur Produktion von medizinisch, pharmakologisch und Nahrungsmittel-relevanten Substanzen.

In der WO 01/00843 ist die Isolierung und der allgemeine Einsatz zur Herstellung von Feinchemikalien einer Vielzahl von Genen aus *Corynebacterium glutamicum*, die für Proteine des Zentralstoffwechsels kodieren, offenbart. Ein Verfahren zur Herstellung und verbesserten Aufbereitung der Aminosäure L-Serin wird dagegen nicht beschrieben.

Neben der Biosynthese von wirtschaftlich interessanten L-Aminosäuren, wie z. B. L-Serin ist auch die Sekretion dieser Stoffwechselprodukte in das Kulturmedium von entscheidender Bedeutung für die Ausbeute an L-Serin im Endprodukt. Diese Ausschleusung kann unspezifisch durch Diffusion erfolgen oder wie es beispielsweise für die Aminosäuren L-Isoleucin oder L-Lysin beschrieben ist (Zittrich, S. et al., 1994, Journal of Bacteriology, 176: 6892-6899 und Bröer, S. et al., 1991, European Journal of Biochemistry, 202: 131-153), aktiv durch Membrantransportsysteme vermittelt werden. Problematisch bei solchen aktiven Transportsystemen ist, daß die Kapazität dieser "Exportcarrier" schnell überschritten wird, sobald der Gehalt an zu transportierendem Stoffwechselprodukt in der Zelle einen kritischen Wert der natürlicher Weise vorliegenden Konzentration überschreitet. D. h., daß beispielsweise bei einer gesteigerten Biosynthese an L-Serin, dessen Ausschleusung aus der Zelle limitiert sein kann.

Folglich ist die Verfügbarkeit der entscheidend an der Biosynthese von L-Serin beteiligten Gene aus coryneformen Bakterien zur Expression in einem homologen System wünschenswert ebenso wie eine verbesserte Sekretion des gebildeten L-Serin in das Kulturmedium.

Ziel der vorliegenden Erfindung ist es daher, ein System zur Verfügung zu stellen, das die zuvor genannten Nachteile nicht mehr aufweist und eine verbesserte Produktion von L-Serin oder davon ableitbaren Stoffwechselprodukten und deren Isolierung ermöglicht.

Diese Aufgabe wird durch ein Verfahren zur mikrobiellen Herstellung von L-Serin gelöst, bei welchem (a) eine Nukleinsäure kodierend für eine Phosphoserin-Phosphatase (serB) gemäß SEQ ID No. 1 oder 5 und eine Nukleinsäure kodierend für eine Phosehoserin-Aminotransferase (serC) gemäß SEQ ID No. 3 oder 7 und eine Nukleinsäure kodierend für einen L-Threoninexportcarrier gemäß SEQ ID No. 9 oder 11 isoliert aus einem coryneformen Bakterium in einen homologen Mikroorganismus übertragen und dort exprimiert werden, wobei die Expression und die Aktivität der entsprechend kodierten Polypeptide gegenüber dem entsprechend nicht genetisch veränderten Mikroorganismus erhöht sind, (b) dieser genetisch veränderte Mikroorganismus aus Schritt a) zur fermentativen Herstellung von L-Serin eingesetzt wird, wobei L-Serin verstärkt in das Kulturmedium sezerniert wird und (c) das entsprechend gebildete L-Serin aus dem Kulturmedium isoliert wird.

Außerdem wird die Aufgabe durch einen genetisch veränderten Mikroorganismus zur Herstellung von L-Serin enthaltend in replizierbarer Form eine Nukleinsäure kodierend für eine Phosphoserin-Phosphatase (serB) gemäß SEQ ID No. 1 oder 5 und eine Nukleinsäure kodierend für eine Phosphoserin-Aminotransferase (serC) gemäß SEQ ID No 3 oder 7 und eine Nukleinsäure kodierend für den L-Threoninexportcarrier (thrE) gemäß SEO ID No 9 oder 11, welche im Vergleich zu dem entsprechend nicht genetisch veränderten Mikroorganismus verstärkt exprimiert werden und/oder deren Kopienzahl erhöht ist, gelöst.

Erfindungsgemäß umfaßt ist auch die Verwendung der Nukleinsäuren kodierend für einen L-Threonin-Exportcarrier gemäß SEQ ID No 9 oder 11 sowie die davon abgeleiteten Polypeptidsequenzen gemäß SEQ ID No 10 oder 12 sowie deren Einsatz in die erfindungsgemäßen Verfahren zur Herstellung von L-Serin. Die Isolierung dieser Sequenzen aus coryneformen Bakterien ist in der deutschen Patentanmeldung 199 41 478.5 offenbart.

Die eingesetzten Nukleinsäuren zeichnen sich dadurch aus, daß sie aus coryneformen Bakterien, bevorzugt der Gattung *Corynebacterium* oder *Brevibacterium*, besonders bevorzugt der Art *Corynebacterium glutamicum* oder *Brevibacterium flavum* isoliert werden. Beispiele für in Stammkulturen hinterlegte Wildtypen coryneformer Bakterien sind *Corynebacterium glutamicum* ATCC 13032, *Corynebacterium glutamicum* ATCC 14752, *Corynebacterium acetoglutamicum* ATCC 15806, *Corynebacterium acetoglutamicum* ATCC 15806, *Corynebacterium melassecola* ATCC 17965, *Corynebacterium thermoaminogenes* FERM BP-1539, *Brevibacterium flavum* ATCC 14067, *Brevibacterium lactofermentum* ATCC 13869 und *Brevibacterium divaricatum* ATCC 14020. Beispiele für zur Herstellung von L-Serin geeignete Mutanten oder Produktionsstämme sind *Corynebacterium glutamicum* ATCC 21586, *Corynebacterium glutamicum* KY 10150 und *Brevibacterium ketoglutamicum* ATCC 21222. Die vorliegende Erfindung wird durch die Angabe der zuvor genannten Bakterienstämme näher charakterisiert, die jedoch nicht limitierend wirkt.

Unter einer isolierten Nukleinsäure oder einem isolierten Nukleinsäurefragment ist erfindungsgemäß ein Polymer aus RNA oder DNA zu verstehen, das einzel- oder doppelsträngig sein kann und optional natürliche, chemisch synthetisierte, modifizierte oder artifizielle Nukleotide enthalten kann. Der Begriff DNA-Polymer schließt hierbei auch genomische DNA, cDNA oder Mischungen davon ein.

Unter Allelen sind erfindungsgemäß funktionell äquivalente, d. h. im wesentlichen gleichwirkende Nukleotidsequenzen zu verstehen. Funktionell äquivalente Sequenzen sind solche Sequenzen, welche trotz abweichender Nukleotidsequenz, beispielsweise durch die Degeneriertheit des genetischen Codes bedingt noch die gewünschten Funktionen besitzen. Funktionelle Äquivalente umfassen somit natürlich vorkommende Varianten der hierin beschriebenen Sequenzen sowie künstliche, z. B. durch chemische Synthese erhaltene und gegebenenfalls an den Kodon-Gebrauch des Wirtsorganismus angepaßte NukleotidSequenzen. Darüber hinaus umfassen funktionell äquivalente Sequenzen solche, die eine veränderte Nukleotidsequenz aufweisen, welche dem Enzym beispielsweise eine Desensitivität oder Resistenz gegenüber Inhibitoren verleiht.

Unter einem funktionellen Äquivalent versteht man insbesondere auch natürliche oder künstliche Mutationen einer ursprünglich isolierten Sequenz, welche weiterhin die gewünschte Funktion zeigen. Mutationen umfassen Substitutionen, Additionen, Deletionen, Vertauschungen oder Insertionen eines oder mehrerer Nukleotidreste.
Inbegriffen sind hier auch sogenannte Sinnmutationen (sense mutations), die auf Proteinebene beispielsweise zum Austausch konservierter Aminosäuren führen können, welche aber zu keiner grundsätzlichen Veränderung der Aktivität des Proteins führen und somit funktionsneutral sind. Dies beinhaltet auch Veränderungen der Nukleotidsequenz, die auf Proteinebene den N- oder C-Terminus eines Proteins betreffen, ohne jedoch die Funktion des Proteins wesentlich zu beeinträchtigen. Diese Veränderungen können sogar stabilisierenden Einfluß auf die Proteinstruktur ausüben.

Ferner werden beispielsweise auch solche Nukleotidsequenzen durch die vorliegende Erfindung mit umfaßt, welche man durch Modifikation der Nukleotidsequenz, resultierend in entsprechenden Derivaten, erhält. Ziel einer solchen Modifikation kann z. B. die weitere Eingrenzung der darin enthaltenen kodierenden Sequenz oder z. B. auch die Einfügung weiterer Restriktionsenzym-Schnittstellen sein. Funktionelle Äquivalente sind auch solche Varianten, deren Funktion, verglichen mit dem Ausgangsgen bzw. Genfragment, abgeschwächt oder verstärkt ist.

Außerdem sind artifizielle DNA-Sequenzen Gegenstand der vorliegenden Erfindung, solange sie, wie oben beschrieben, die gewünschten Eigenschaften vermitteln. Solche artifiziellen DNA-Sequenzen können beispielsweise durch Rückübersetzung von mittels computergestützten Programmen (molecular modelling) erstellten Proteinen oder durch invitro-Selektion ermittelt werden. Besonders geeignet sind kodierende DNA-Sequenzen, die durch Rückübersetzung einer Polypeptidsequenz gemäß der für den Wirtsorganismus spezifischen Kodon-Nutzung erhalten wurden. Die spezifische Kodon-Nutzung kann ein mit molekulargenetischen Methoden vertrauter Fachmann durch Computerauswertungen anderer, bereits bekannter Gene des zu transformierenden Organismus leicht ermitteln.

Unter homologen Sequenzen sind erfindungsgemäß solche zu verstehen, die zu den erfindungsgemäßen Nukleotidsequenzen komplementär sind und/oder mit diesen hybridisieren. Der Begriff hybridisierende Sequenzen schließt erfindungsgemäß substanziell ähnliche Nukleotidsequenzen aus der Gruppe von DNA oder RNA ein, die unter an sich bekannten stringenten Bedingungen eine spezifische Wechselwirkung (Bindung) mit den zuvor genannten Nukleotidsequenzen eingehen. Hierzu zählen auch kurze Nukleotidsequenzen mit einer Länge von beispielsweise 10 bis 30, bevorzugt 12 bis 15 Nukleotiden. Dies umfaßt erfindungsgemäß u. a. auch sogenannte Primer oder Sonden.

Erfindungsgemäß sind auch die den kodierenden Bereichen (Strukturgenen) vorausgehenden (5'- oder upstream) und/oder nachfolgenden (3'- oder downstream) Sequenzbereiche eingeschlossen. Insbesondere sind hierin Sequenzbereiche mit regulatorischer Funktion inbegriffen. Sie können die Transkription, die RNA-Stabilität oder das RNA processing sowie die Translation beeinflussen. Beispiele für regulatorische Sequenzen sind u. a. Promotoren, Enhancer, Operatoren, Terminatoren oder Translationsverstärker.

Gegenstand der vorliegenden Erfindung ist ferner eine Genstruktur enthaltend wenigstens eine der zuvor beschriebenen Nukleotidsequenzen kodierend für eine Phosphoserin-Phosphatase, eine Phosphoserin-Aminotransferase und/oder einen L-Threoninexportcarrier sowie mit diesen operativ verknüpfte regulatorische Sequenzen, welche die Expression der kodierenden Sequenzen in der Wirtszelle steuern.

Unter einer operativen Verknüpfung versteht man die sequenzielle Anordnung beispielsweise von Promotor, kodierender Sequenz, Terminator und ggf. weiterer regulatorischer Elemente derart, daß jedes der regulatorischen Elemente seine Funktion bei der Expression der kodierenden Sequenz bestimmungsgemäß erfüllen kann. Diese regulatorischen Nukleotidsequenzen können natürlichen Ursprungs sein oder durch chemische Synthese erhalten werden. Als Promotor ist grundsätzlich jeder Promotor geeignet, der die Genexpression in dem entsprechenden Wirtsorganismus steuern kann. Hierbei kann es sich erfindungsgemäß auch um einen chemisch induzierbaren Promotor handeln, durch den die Expression der ihm unterliegenden Gene in der Wirtszelle zu einem bestimmten Zeitpunkt gesteuert werden kann. Beispielhaft sei hier ein durch IPTG (Isopropyl-β-thiogalactosid) induzierbarer Promotor genannt (Eikmanns, B. J. et al., 1991, Gene, 102:93-98).

Die Herstellung einer Genstruktur erfolgt durch Fusion eines geeigneten Promotors mit wenigstens einer erfindungsgemäßen Nukleotidsequenz nach gängigen Rekombinations- und Klonierungstechniken, wie sie beispielsweise in T. Maniatis, E.F. Fritsch und J. Sambrook, Molecular Cloning: A Laboratory Manual, Cold Spring Harbor Laboratury, Cold Spring Harbor, NY (1989) beschrieben sind.
Für die Verbindung der DNA-Fragmente miteinander können an die Fragmente Adaptoren oder Linker angesetzt werden.

Darüber hinaus kann im erfindungsgemäßen Verfahren ein Vektor enthaltend wenigstens eine Nukleotidsequenz der zuvor beschriebenen Art kodierend für eine Phosphoserin-Phosphatase, eine Phosphoserin-Aminotransferase und/oder einen L-Threoninexportcarrier, mit diesen operativ verknüpfte regulative Nukleotidsequenzen sowie zusätzliche Nukleotidsequenzen zur Selektion transformierter Wirtszellen, für die Replikation innerhalb der Wirtszelle oder zur Integration in das entsprechende Wirtszell-Genom, kann im erfindungsgemäßen Verfahren verwendet werden Ferner kann der Vektor eine Genstruktur der vorgenannten Art enthalten.

Als Vektoren eignen sich solche, die in coryneformen Bakterien repliziert werden (Process Biochem 33 (1998) 147-161). Zahlreiche bekannte Plasmidvektoren wie z. B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z. B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden. Diese Aufzählung ist für die vorliegende Erfindung jedoch nicht limitierend.

Unter Ausnutzung der Nukleinsäuresequenzen gemäß der SEQ ID No 1, 3, 5, 7, 9 oder 11 können entsprechende Sonden oder auch Primer synthetisiert und dazu verwendet werden, beispielsweise mit Hilfe der PCR-Technik analoge Gene aus anderen Mikroorganismen, bevorzugt coryneformen Bakterien zu amplifizieren und zu isolieren.

Gegenstand der vorliegenden Erfindung ist somit auch eine Sonde zur Identifizierung und/oder Isolierung von Genen kodierend für an der Biosynthese von L-Serin oder am Export von L-Threonin und/oder L-Serin beteiligte Proteine, wobei diese Sonde ausgehend von den Nukleinsäuresequenzen der zuvor beschriebenen Art hergestellt wird und eine zur Detektion geeignete Markierung enthält. Bei der Sonde kann es sich um einen Teilausschnitt der erfindungsgemäßen Sequenz, beispielsweise aus einem konservierten Bereich handeln, der z. B. eine Länge von 10 bis 30 oder bevorzugt 12 bis 15 Nukleotiden aufweist und unter stringenten Bedingungen spezifisch mit homologen Nukleotidsequenzen hybridisieren kann. Geeignete Markierungen sind aus der Literatur zahlreich bekannt.

Anleitungen hierzu findet der Fachmann unter anderem beispielsweise im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994) oder beispielsweise im Handbuch "The DIG System Users Guide for Filter Hybridization" der Firma Roche Diagnostics (Mannheim, Deutschland) und bei Liebl et al. (International Journal of Systematic Bacteriology (1991) 41: 255-260).

Gegenstand der vorliegenden Erfindung ist ferner eine Phosphoserin-Phosphatase oder ein Teil davon kodiert durch eine Nukleinsäuresequenz ausgewählt aus den Sequenzen gemäß den SEQ ID No 1 oder 5 oder deren Variationen der zuvor beschriebenen Art. Die vorliegende Erfindung umfaßt ebenso eine Phosphoserin-Phosphatase mit einer Aminosäuresequenz ausgewählt aus den Sequenzen gemäß der SEQ ID No 2 oder 6 oder einer modifizierten Form dieser Polypeptidsequenzen oder Isoformen davon oder Mischungen daraus.

Ebenso ist eine Phosphoserin-Aminotransferase oder ein Teil davon kodiert durch eine Nukleinsäuresequenz ausgewählt aus den Sequenzen gemäß den SEQ ID No 3 oder 7 oder deren Variationen der zuvor beschriebenen Art umfaßt. Die vorliegende Erfindung betrifft ebenso eine Phosphoserin-Phosphatase mit einer Aminosäuresequenz ausgewählt aus den Sequenzen gemäß der SEQ ID No 4 oder 8 oder einer modifizierten Form dieser Polypeptidsequenzen oder Isoformen davon oder Mischungen daraus.

Die vorliegende Erfindung schließt auch die Verwendung des L-Threonin-Exportcarriers mit einer Aminosäuresequenz ausgewählt aus den Sequenzen gemäß SEQ ID No 10 oder 12 oder einer modifizierten Form dieser Polypeptidsequenzen oder Isoformen davon oder Mischungen daraus ein, wobei der Carrier auch den Transport von L-Serin vermittelt und durch eine Nukleinsäuresequenz ausgewählt aus den Sequenzen gemäß SEQ ID No 9 oder 11 oder deren Variationen kodiert wird

Die erfindungsgemäßen Polypeptide zeichnen sich ferner dadurch aus, daß sie aus coryneformen Bakterien, bevorzugt der Gattung *Corynebacterium* oder *Brevibacterium*, besonders bevorzugt der Art *Corynebacterium glutamicum* oder *Brevibacterium flavum* stammen.

Unter Isoformen sind Enzyme mit gleicher oder vergleichbarer Substrat-und Wirkungsspezifität zu verstehen, die jedoch eine unterschiedliche Primärstruktur aufweisen.

Unter modifizierten Formen sind erfindungsgemäß Enzyme zu verstehen, bei denen Änderungen in der Sequenz, beispielsweise am N- und/oder C-Teminus des Polypeptids oder im Bereich konservierter Aminosäuren vorliegen, ohne jedoch die Funktion des Enzyms zu beeinträchtigen. Diese Veränderungen können in Form von Aminosäureaustauschen nach an sich bekannten Methoden vorgenommen werden.

Eine besondere Ausführungsvariante der vorliegenden Erfindung umfaßt Varianten der erfindungsgemäßen Polypeptide, deren Aktivität beispielsweise durch Aminosäureaustausche, verglichen mit dem jeweiligen Ausgangsprotein, abgeschwächt oder verstärkt ist. Gleiches gilt für die Stabilität der erfindungsgemäßen Enzyme in den Zellen, die beispielsweise gegenüber dem Abbau durch Proteasen verstärkt oder vermindert anfällig sind.

Ferner sind Polypeptide mit der Funktion einer Phosphoserin-Phosphatase oder Phosphoserin-Aminotransferase Gegenstand der vorliegenden Erfindung, die in ihrer Aminosäuresequenz derart verändert sind, daß sie gegenüber regulatorisch wirkenden Verbindungen, beispielsweise die sie in ihrer Aktivität regulierenden Stoffwechsel-Endprodukte desensitiv sind (feedback-desensitiv).

Gegenstand der vorliegenden Erfindung ist ferner die Übertragung wenigstens einer der Nukleinsäuresequenzen oder eines Teils davon kodierend für eine Phosphoserin-Phosphatase oder Phosphoserin-Aminotransferase, ein Allel, Homolog oder Derivat davon gemäß SEQ ID No 1, 3, 5 oder 7 sowie einer Nukleinsäuresequenz kodierend für einen L-Threoninexportcarrier ein Allel, Homolog oder Derivat davon gemäß SEQ ID No 9 oder 11 in ein homologes Wirtssystem. Dies schließt auch die Übertragung eines zuvor beschriebenen Genkonstrukts oder Vektors in ein homologes Wirtssystem ein. Diese Übertragung von DNA in eine Wirtzelle erfolgt nach gentechnischen Methoden. Als bevorzugtes Verfahren sei hier die Transformation und besonders bevorzugt die Übertragung von DNA durch Elektroporation genannt.

Unter einem homologen Wirtssystem sind Mikroorganismen zu verstehen, die alle einer verwandten Familie angehören. Erfindungsgemäß sind hierunter coryneforme Bakterien zu verstehen, in die die erfindungsgemäß aus coryneformen Bakterien isolierten Nukleinsäuren eingebracht werden. Ein aus einer erfolgreich durchgeführten Nukleinsäureübertragung resultierender transformierter Mikroorganismus unterscheidet sich somit von dem entsprechend nicht transformierten Mikroorganismus dadurch, daß er zusätzliche Nukleinsäuren der erfindungsgemäßen Art enthält und entsprechend zur Ausprägung bringen kann. Stellvertretend für ein geeignetes homologes Wirtssystem sei das Bakterium *Corynebacterium glutamicum* und bevorzugt der Stamm ATCC 13032 genannt, welches unter Standardbedingungen wie folgt kultiviert werden kann:
Die Kultivierung erfolgt in 500 ml Schüttelkolben bei 120 Umdrehungen/min und 30°C, wobei pro Kolben 50 ml Kulturmedium eingesetzt werden. Die Beimpfung des Kulturmediums erfolgt durch Zugabe einer Bakterien-(Vor)-Kultur des Stammes *Corynebacterium glutamicum* ATCC 13032, der zuvor unter gleichen Bedingungen über einen Zeitraum von 12-16 Stunden gezüchtet wurde, wobei eine optische Dichte der beimpften Kulturmedium im Bereich von 0,7 bis 1,5 eingestellt wird.
Als Kulturmedium ist je nach Anforderungen ein Komplexmedium, wie z. B. LB-Medium (T. Maniatis, E.F. Fritsch und J. Sambrook, Molecular Cloning: A Laboratory Manual, Cold Spring Harbor Laboratury, Cold Spring Harbor, NY (1989)) oder auch ein Mineralsalzmedium, wie z. B. CGXII-Medium (Keilhauer, C. et al., 1993, J. Bacteriol., 175:5593-5603) geeignet. Nach entsprechender Kultivierung kann die Bakteriensuspension geerntet und zur weiteren Untersuchung, beispielsweise zur Transformation oder zur Isolierung von Nukleinsäuren nach gängigen Methoden eingesetzt werden.
Diese Vorgehensweise kann analog auch auf andere coryneforme Bakterienstämme angewendet werden. Dabei werden als Wirtssysteme Bakterien der Gattung *Corynebacterium* oder -*Brevibacterium* bevorzugt. Innerhalb der Gattung *Corynebacterium* wird besonders die Art *Corynebacterium glutamicum* und innerhalb der Gattung *Brevibacterium* besonders die Art *Brevibacterium flavum* bevorzugt. Zu den Vertretern dieser Gattungen zählen zum einen Stämme, die in ihren Eigenschaften als Wildtyp charakterisiert sind. Hier sind beispielsweise *Corynebacterium glutamicum* ATCC 13032, *Corynebacterium glutamicum* ATCC 14752, *Corynebacterium acetoglutamicum* ATCC 15806, *Corynebacterium acetoglutamicum* ATCC 15806, *Corynebacterium melassecola* ATCC 17965, *Corynebacterium thermoaminogenes* FERM BP-1539, *Brevibacterium flavum* ATCC 14067, *Brevibacterium lactofermentum* ATCC 13869 und *Brevibacterium divaricatum* ATCC 14020 zu nennen.

Darüber hinaus schließt die vorliegende Erfindung auch Bakterienstämme ein, die sich als L-Serin produzierende Mutanten oder Produktionsstämme auszeichnen. Diese können z. B. ausgehend von Wildtypstämmen durch klassische (chemische oder physikalische) oder gentechnische Methoden hergestellt werden. Beispiele für erfindungsgemäß geeignete Stämme sind u. a. *Corynebacterium glutamicum* ATCC 21586, *Corynebacterium glutamicum* KY 10150 und *Brevibacterium ketoglutamicum* ATCC 21222. Die vorliegende Erfindung wird durch die ausgewählten Beispiele an Mikroorganismen näher charakterisiert, jedoch nicht limitiert.

Erfindungsgemäß umfaßt sind neben den zuvor beschriebenen Bakterienstämmen, die sich als L-Serin-Produzenten auszeichnen, auch solche Produktionsstämme, die eine verbesserte Sekretion der gewünschten Stoffwechselprodukte, bevorzugt von L-Aminosäuren, aus den Zellen in das Kulturmedium aufweisen. Diese verbesserte Sekretion kann beispielsweise durch eine Überexpression eines oder mehrerer Gene kodierend für Membrantransportproteine, beispielsweise Exportcarrier-Proteine, u.a. spezifische L-Aminosäure-Exportcarrier-Proteine, erreicht werden.

In einer besonderen Ausführungsvariante der vorliegenden Erfindung zeichnet sich der zur L-Serinproduktion eingesetzte Bakterienstamm dadurch aus, daß er ein genetisch veränderter Mikroorganismus ist, enthaltend in replizierbarer Form eine Nukleinsäure kodierend für eine Phosphoserin-Phosphatase (serB) und/oder eine Nukleinsäure kodierend für eine Phosphoserin-Aminotransferase (serC) gemäß SEQ ID No 1, 3 ,5 oder 7 und eine Nukleinsäure kodierend für den L-Threoninexportcarrier (thrE) gemäß SEQ ID No 9 oder 11, welche im Vergleich zu dem entsprechend nicht genetisch veränderten Mikroorganismus verstärkt exprimiert werden und/oder deren Kopienzahl erhöht ist.

Ebenso ist ein erfindungsgemäß ein genetisch veränderter Mikroorganismus umfaßt, enthaltend Polypeptide kodiert durch die Gene serB und/oder serC gemäß SEQ ID No 2, 4, 6 oder 8 und thrE gemäß SEQ ID No 10 oder 12, welche eine erhöhte Aktivität und/oder Lebensdauer und/oder eine geringere Endprodukt-Hemmung im Vergleich zu dem entsprechend nicht genetisch veränderten Mikroorganismus aufweisen. Somit ist ebenfalls ein genetisch veränderter Mikroorganismus Gegenstand der vorliegenden Erfindung, der wenigstens eine erhöhte L-Serinproduktionsrate und zusätzlich eine erhöhte L-Serin- und/oder L-Threonin-Sekretionsrate aufweist.

Ebenso umfaßt die vorliegende Erfindung einen genetisch veränderten Mikroorganismus enthaltend in replizierbarer Form eine Genstruktur oder einen Vektor der zuvor beschriebenen Art. Ein erfindungsgemäß genetisch veränderter Mikroorganismus zeichnet sich ferner dadurch aus, daß er ein coryneformes Bakterium, bevorzugt der Gattung *Corynebacterium* oder *Brevibacterium*, besonders bevorzugt der Spezies *Corynebacterium glutamicum* oder *Brevibacterium flavum* ist.

Prinzipiell können Gene durch an sich bekannte Methoden, wie beispielsweise die Polymerase-Ketten-Reaktion (PCR) mit Hilfe von kurzen, synthetischen Nukleotidsequenzen (Primern) amplifiziert und anschließend isoliert werden. Die Herstellung der verwendeten Primer erfolgt im Allgemeinen anhand bekannter Gensequenzen aufgrund bestehender Homologien in konservierten Bereichen der Gene und/oder unter Berücksichtigung des GC-Gehalts der DNA des zu untersuchenden Mikroorganismus. Diese Methode weist jedoch eine Reihe von Nachteilen auf, die beispielsweise in der Fehlerhaftigkeit der PCR-Methode selbst begründet liegen oder darin, daß die zu identifizierenden Gensequenzen geringere Homologien zu den bereits bekannten Sequenzen aufweisen als anzunehmen ist. Dies kann zu einer unspezifischen oder sogar ausbleibenden Hybridisierung der eingesetzten Primer an die zu untersuchende Nukleinsäuresequenz führen.

Eine weitere Vorgehensweise zur Isolierung von kodierenden Nukleotidsequenzen ist die Komplementation von sogenannten DefektMutanten des zu untersuchenden Organismusses, die zumindest phänotypisch einen Funktionsverlust in der Aktivität des zu untersuchenden Gens oder entsprechenden Proteins aufweisen. Unter einer Komplementation ist die Aufhebung des Gendefektes der Mutante und weitgehende Wiederherstellung des ursprünglichen Erscheinungsbildes vor der Mutatgenese zu verstehen, die durch die Einbringung funktioneller Gene oder Genfragmente aus dem zu untersuchenden Mikroorganismus erreicht wird.

Ein klassisches Mutagenese-Verfahren zur Herstellung von Defektmutanten ist beispielsweise die Behandlung der Bakterienzellen mit Chemikalien wie z. B. N-Methyl-N-Nitro-N-Nitrosoguanidin oder UV-Bestrahlung. Derartige Verfahren zur Mutationsauslösung sind allgemein bekannt und können unter anderem bei Miller (A Short Course in Bacterial Genetics, A Laboratory Manual and Handbook for *Escherichia coli* and Related Bacteria (Cold Spring Harbor Laboratory Press, 1992)) oder im Handbuch "Manual of Methods for General Bacteriology" der American Society for Bacteriology (Washington D.C., USA, 1981) nachgelesen werden. Nachteilig ist hierbei eine zeit- und kostenintensive Selektion der Mutanten mit dem gewünschten Phänotyp sowie die Tatsache, daß die isolierten Mutanten genetisch undefiniert sind, da die Mutagenese ungerichtet erfolgt. Letzeres führt häufig zu unerwarteten Problemen, beispielsweise hinsichtlich der Stabilität dieser Mutanten im Rahmen eines Produktionsverfahrens im großtechnischen Maßstab.

Eine weitere Aufgabe der vorliegenden Erfindung ist es, ein Verfahren zur Isolierung von kodierenden Nukleinsäuresequenzen aus coryneformen Bakterien zur Verfügung zu stellen, welches die genannten Nachteile nicht mehr aufweist. Die erfindungsgemäße Lösung wird durch die nachfolgende Beschreibung näher erläutert.

Die Erfindung betrifft ein Verfahren zur Isolierung der erfindungsgemäßen Nukleinsäuren, wobei ein coryneformes Bakterium erzeugt wird, welches durch Transposon-Mutagenese erzeugte Defekte in den Genen *serB* und *serC* aufweist.

Bei der Methode der Transposon-Mutagenese wird die Eigenschaft eines Transposons ausgenutzt, welches in DNA-Sequenzen zu "springen" und dadurch die Funktion des betreffenden Gens zu stören bzw. auszuschalten vermag.

Beispiele für Transposons coryneformer Bakterien sind nachfolgend aufgeführt. So wurde aus dem *Corynebacterium* xerosis Stamm M82B das Erythromycinresistenz-Transposon Tn5432 (Tauch et al., Plasmid (1995) 33: 168-179) und das Chloramphenicolreistenz-Transposon Tn5546 isoliert. Tauch et al. (Plasmid (1995) 34: 119-131 und Plasmid (1998) 40: 126-139) zeigten, daß eine Mutagenese mit diesen Transposons möglich ist. Weiterhin wurde aus *Corynebacterium glutamicum* ATCC 31831 die Insertionssequenz IS31831 isoliert (Vertes et al., Molecular Microbiology (1994) 11: 739-746). Durch Kombination von IS31831 mit dem Kanamycinresistenzgen aphA wurde das künstliche Transposon Tn31831 konstruiert (Vertes et al., Molecular and General Genetics (1994) 245: 397-405). Vertes et al. (Molecular and General Genetics (1994) 245: 397-405) und Jaeger et al. (FEMS Microbiology Letters (1995) 126: 1-6) demonstrierten die Anwendung dieser Transposons bei den Stämmen *Brevibacterium flavum* MJ233C und *Corynebacterium glutamicum* ATCC 13058.

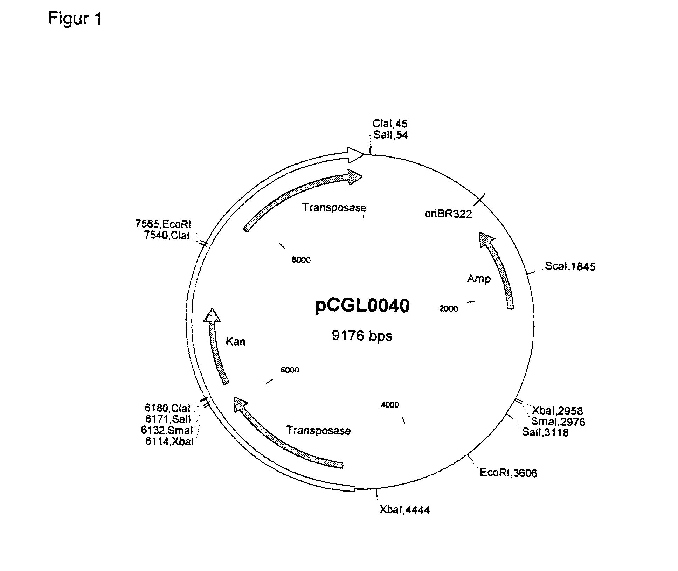

Ein anderes Transposon ist das Transposon Tn5531, das bei Ankri et al. (Journal of Bacteriology (1996) 178: 4412-4419) beschrieben wird und beispielhaft im Laufe der vorliegenden Erfindung eingesetzt wird. In einer besonderen Ausführungsvariante der vorliegenden Erfindung wird hierzu der *Corynebacterium glutamicum* Stamm ATCC 14752 einer entsprechenden Mutagenese unterworfen. Wahlweise eignet sich auch der *Corynebacterium glutamicum* Stamm ATCC 13032. Das Transposon Tn5531 enthält das aph3 Kanamycinresistenzgen und kann in Form des Plasmidvektors pCGL0040 (Figur 1) verabreicht werden. Die Nukleotidsequenz des Transposons Tn5531 ist unter der Zugangsnummer (accession number) U53587 bei dem National Center for Biotechnology Information (NCBI, Bethesda, MD, USA) frei verfügbar. Die vorliegende Erfindung wird durch die Aufzählung der zuvor genannten Transposons näher charakterisiert, jedoch nicht limitiert.

Im Anschluß an die Transposon-Mutagenese wird eine in dem/n gewünschten Gen/en defekte Mutante selektioniert. Eine im *serB*-und/oder *serC*-Gen defekte Mutante wird erfindungsgemäß daran erkannt, daß sie auf Minimalmedium mit L-Serin gutes Wachstum aber auf Minimalmedium ohne L-Serin jedoch schlechtes Wachstum zeigt.

Die entsprechend selektionierten Dekfektmutanten coryneformer Bakterienstämme werden anschließend für die Klonierung und Sequenzierung des *serB*-, und *serC*-Gens eingesetzt.

Die Klonierung der Gene kann beispielsweise durch Komplementation der Defektmutanten erfolgen. Hierzu wird eine Genbank von der DNA des zu untersuchenden coryneformen Bakteriums angelegt. Das Anlegen von Genbanken ist in allgemein bekannten Lehrbüchern und Handbüchern niedergeschrieben. Als Beispiele sind das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Bathe et al. (Molecular and General Genetics, 252:255-265, 1996) beschreiben eine Genbank von *Corynebacterium glutamicum* ATCC 13032, die mit Hilfe des Cosmidvektors SuperCos I (Wahl et al., 1987, Proceedings of the National Academy of Sciences USA, 84:2160-2164) im *E. coli* K-12 Stamm NM554 (Raleigh et al., 1988, Nucleic Acids Research 16:1563-1575) angelegt wurde. Erfindungsgemäß eignen sich solche Vektoren, die in coryneformen Bakterien, vorzugsweise *Corynebacterium glutamicum*, replizieren. Derartige Vektoren sind aus dem Stand der Technik bekannt. Als Beispiel sei der Plasmidvektor pZ1 genannt, der bei Menkel et al. (Applied and Environmental Microbiology (1989) 64: 549-554) beschrieben ist.

Die Genbank wird anschließend mittels Transformation, erfindungsgemäß bevorzugt durch Elektroporation in den im *serB-* oder *serC*-Gen defekten und zuvor beschriebenen Bakterienstamm überführt. Nach an sich bekannten Methoden werden diejenigen transformierten Bakterien ausgewählt, die die Fähigkeit besitzen in Abwesenheit von L-Serin auf Minimalmedium zu wachsen. Die aus diesen selektionierten Transformanden re-isolierten DNA-Fragmente der ursprünglich eingesetzten Genbank können anschließend einer Sequenzanalyse unterzogen werden.

In einer weiteren Ausführungsvariante der vorliegenden Erfindung kann aufgrund der durch Mutagenese mit dem Transposon Tn5531 erzeugten Defektmutanten eines coryneformen Bakteriums wie z. B. den Stämmen ATCC 14752*serB*::Tn5531 und ATCC 14752*serC*::Tn5531 das *serB*::Tn5531-Allel bzw. das *serC*::Tn5531-Allel direkt unter Ausnutzung des in dem Transposon enthaltenen Kanamycinresistenzgens aph3 kloniert und isoliert werden. Hierzu verwendet man bekannte Klonierungs-Vektoren wie z. B. pUC18 (Norrander et al., Gene (1983) 26: 101-106 und Yanisch-Perron et al., Gene (1985) 33: 103-119) oder pGEM-T (Zhou M-Y, Clark SE und Gomez-Sanchez CE (1995) BioTechniques 19: 34; Kobs G (1995) Promega Notes 55: 28; Promega Cooperation, Madison, USA). Als Wirtssysteme zur Klonierung eignen sich besonders solche *Escherichia coli*-Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die Selektion auf Transformanden erfolgt in Gegenwart von Kanamycin.

Die aus den erhaltenen Transformanden isolierte DNA, welche die interessierenden Gene enthält, wird anschließend sequenziert. Hierzu kann die von Sanger et al. (Proceedings of the National Academy of Sciences of the United States of America USA (1977) 74: 5463-5467) beschriebene Dideoxy-Kettenabbruchmethode verwendet werden. Hiernach erhält man die stromaufwärts und stromabwärts des Tn5531-Insertionsortes enthaltenen Gene. Die erhaltenen Nukleotidsequenzen werden dann mit kommerziell erhältlichen Sequenzanalyse-Programmen wie z. B. dem Programmpaket Lasergene (Biocomputing Software for Windows, DNASTAR, Madison, USA) oder dem Programmpaket HUSAR (Release 4.0, EMBL, Heidelberg, Deutschland) analysiert und zusammengefügt. Auf die zuvor beschriebene Weise wurden die erfindungsgemäßen Nukleinsäuren des *serB-* und serC-Gens coryneformer Bakterien isoliert und deren Sequenz bestimmt.

Überraschender Weise haben Homologievergleiche mit bekannten Sequenzen ergeben, daß das von *serB* kodierte Polypeptid eine moderate Ähnlichkeit zu der Phosphoserin-Phosphatase aus *Escherichia coli* aufweist, während das von *serC* abgeleitete Polypeptid nur geringe, wenn auch signifikante Ähnlichkeit zu der Phosphoserin-Aminotransferase von *Escherichia coli* aufweist. Auch zu bekannten Phosphoserin-Phosphatasen bzw. Phosphoserin-Aminotransferasen aus anderen Organismen (z. B. Hefe) weisen die gefundenen corynebacteriellen Gene nur geringe bis moderate Ähnlichkeit auf. Lediglich die abgeleiteten Polypeptidsequenzen der mutmaßlichen *serB*- und *serC*-Gene aus *Mycobacterium tuberculosis* zeigen hohe Ähnlichkeit zu den corynebacteriellen Proteinen. Das Ergebnis eines solchen Homologievergleichs ist in Tabelle 2 dargestellt. Darüber hinaus ergibt die Nukleotidsequenz der coryneformen Gene, daß die in EP 0 931 833 verwendeten PCR-Primer zwar für die Amplifizierung der Gene aus *Escherichia coli* jedoch aufgrund der besagten geringen Sequenzähnlichkeit nicht für die Isolierung der coryneformen *serB* und *serC* Gene geeignet sind.

Dies verdeutlicht einmal mehr den Vorteil des erfindungsgemäßen Verfahrens der Transposon-Mutagenese zur Klonierung der Gene *serB* und *serC* aus coryneformen Bakterien.

Das erfindungsgemäße Verfahren dient zur mikrobiellen Herstellung von L-Serin, wobei wenigstens eine der erfindungsgemäßen Nukleinsäuren, isoliert aus einem coryneformen Bakterium, in einen homologen Mikroorganismus übertragen und dort exprimiert wird, wobei die Genexpression und/oder die Aktivität des entsprechend kodierten Polypeptids gegenüber dem entsprechend nicht genetisch veränderten Mikroorganismus erhöht ist, dieser genetisch veränderte Mikroorganismus zur fermentativen Herstellung von L-Serin eingesetzt wird und das entsprechend gebildete L-Serin aus dem Kulturmedium isoliert wird.

Weiterhin umfasst die vorliegenden Erfindung Allele und Derivate der SEQ ID no. 1, 3, 5, 7, 9 und 11.

Zur Erzielung einer erhöhten Genexpression (Überexpression) in einem gentechnisch hergestellten Organismus kann die Kopienzahl der entsprechenden Gene erhöht werden. Ferner kann die Promotor-und/oder Regulationsregion und/oder die Ribosomenbindungsstelle, die sich stromaufwärts des Strukturgens befindet, entsprechend so verändert werden, daß die Expression mit erhöhter Rate erfolgt. In gleicher Weise wirken Expressionskassetten, die stromaufwärts des Strukturgens eingebaut werden. Durch induzierbare Promotoren ist es zusätzlich möglich die Expression im Verlaufe der fermentativen L-Serin-Produktion zu steigern. Durch Maßnahmen zur Verlängerung der Lebensdauer der mRNA wird ebenfalls die Expression verbessert. Die Gene oder Genkonstrukte können entweder in Plasmiden mit unterschiedlicher Kopienzahl vorliegen oder im Chromosom integriert und amplifiziert sein. Weiterhin kann auch die Aktivität des Enzyms selbst erhöht sein oder durch die Verhinderung des Abbaus des Enzymproteins verstärkt werden. Alternativ kann ferner eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Die erfindungsgemäß hergestellten genetisch veränderten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Serin kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlenhydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe z. B. Antibiotika hinzugefügt werden. Um aerobe Bedingungen aufrechtzuerhalten werden Sauerstoff oder Sauerstoff haltige Gasmischungen wie z. B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum an L-Serin gebildet hat. Dieses Ziel wird normalerweise innerhalb von 10 Stunden bis 160 Stunden erreicht.

Die Analyse der L-Serin-Bildung kann durch Anionenaustauschchromatographie mit anschließender Ninhydrin Derivatisierung erfolgen so wie bei Spackman et al. (Analytical Chemistry, 30, (1958), 1190) beschrieben, oder sie kann durch reversed phase HPLC erfolgen so wie bei Lindroth et al. (Analytical Chemistry (1979) 51: 1167-1174) beschrieben.

Die Mikroorganismen, die Gegenstand der vorliegenden Erfindung sind, können L-Serin aus Glucose, Saccharose, Lactose, Mannose, Fructose, Maltose, Melasse, Stärke, Cellulose oder aus Glycerin und Ethanol herstellen. Es kann sich um die zuvor bereits näher beschriebenen Vertreter coryneformer Bakterien handeln.

Eine Auswahl an Ergebnissen der Fermentation ist in Tabelle 3 dargestellt. Hierbei zeichnen sich die erfindungsgemäß genetisch veränderten Mikroorganismen durch eine wesentlich verbesserte L-Serin-Produktion gegenüber den entsprechend nicht transformierten Mikroorganismen (Wildtypen) oder den Mikroorganismen aus, die lediglich den Vektor ohne Gen-Insert enthalten.

In einer besonderen Ausführungsvariante der vorliegenden Erfindung ist gezeigt, daß die Überexpression des homologen *serB*-Gens in *C. glutamicum* ATCC 13032 (13032(pVWEx1*serB*)) zu einer wenigstens 4-fachen Steigerung der L-Serin-Akkumulation im Medium im Vergleich zu den Kontrollstämmen führt. Durch die Überexpression des homologen serC-Gens (13032(pVWEx2serC)) kann eine wenigstens 20-fache Steigerung der L-Serin-Akkumulation erreicht werden. Durch die gemeinsame Überexpression beider Gene *serB* und *serC* in einem homologen System ist eine noch weitere Steigerung der L-Serin-Produktion zu erwarten.

Bemerkenswert ist hier vor allen Dingen, daß die Steigerung der L-Serin-Akkumulation bereits mit dem Wildtyp *Corynebacterium glutamicum* ATCC 13032 erreicht wird. Durch den erfindungsgemäßen Einsatz eines homologen Aminosäure-Produktionsstammes kann somit eine noch weiter gesteigerte L-Serin-Produktion erreicht werden.

Unter Aminosäure-Produktionsstämmen sind im Sinne der vorliegenden Erfindung *Corynebacterium glutamicum*-Stämme oder homologe Mikroorganismen zu verstehen, die durch klassische und/oder molekulargenetische Methoden derart verändert sind, daß ihr Stoffwechselfluß verstärkt in die Richtung der Biosynthese von Aminosäuren oder deren Abkömmlingen verläuft (metabolic engineering). Beispielsweise sind bei diesen Aminosäure-Produktionsstämmen ein oder mehrere Gen(e) und/oder die korrespondierenden Enzyme, die an entscheidenden und entsprechend komplex regulierten Schlüsselpositionen des Stoffwechselweges (Flaschenhals) stehen in ihrer Regulation verändert oder sogar dereguliert. Die vorliegende Erfindung umfaßt hierbei sämtliche bereits bekannte Aminosäure-Produktionsstämme, bevorzugt der Gattung *Corynebacterium* oder homologer Organismen. Ferner sind erfindungsgemäß auch diejenigen Produktionsstämme umfaßt, die der Fachmann in Analogie zu Erkenntnissen aus anderen Mikroorganismen, beispielsweise Enterobacterien, Bacillaceen oder Hefe-Arten nach gängigen Methoden herstellen kann.

Ferner sind erfindungsgemäß auch solche Aminosäure-Produktionsstämme umfaßt, bei denen der Abbau von L-Serin verändert, bevorzugt abgeschwächt ist. Dies kann beispielsweise durch gezielte gentechnische Veränderungen an L-Serin degradierenden Enzymen oder den korrespondierenden Genen erfolgen.

Eine weitere Verbesserung der Ausbeute an L-Serin im Endprodukt wird erfindungsgemäß dadurch erreicht, daß die Ausschleusung des L-Serins aus den Zellen in das sie umgebende Medium erheblich verbessert wird. Dies wird erfindungsgemäß durch die verstärkte Expression des L-Threonin-Exportcarriers erreicht, durch den überraschender Weise u.a. auch das gebildete L-Serin aktiv über die Zellmembran transportiert wird. Dadurch wird Gehalt an L-Serin im Kulturmedium noch weiter gesteigert und führt zu einem Endprodukt mit erheblich verbesserter Qualität gegenüber bislang bekannten Produkten.

Erfindungsgemäß sind auch Bakterienstämme umfaßt, die zusätzlich zu den vorteilhaften Eigenschaften hinsichtlich der L-Serin-Produktion über eine verbesserte Fähigkeit der Ausschleusung von L-Serin aus den Zellen in das Kulturmedium verfügen. Bevorzugt sind dabei Bakterien mit einem erhöhten Gehalt an Membrantransportproteinen, wie z.B. einem für L-Aminosäuren spezifischen Exportcarrier, insbesondere dem L-Threonin-Exportcarrier.

Gegenstand der vorliegenden Erfindung ist ferner die Verwendung eines genetisch veränderten Mikroorganismus der zuvor beschriebenen Art zur Herstellung von L-Serin und/oder deren Folgeprodukte nach einem Verfahren der eingangs beschrieben Art.

Die vorliegende Erfindung betrifft ferner die Verwendung der auf zuvor beschriebene Weise hergestellten L-Aminosäuren zum Einsatz in der Nahrungsmittel-, Futtermittel- und/oder Pharmaindustrie oder in der Humanmedizin. Darüber hinaus finden die erfindungsgemäß hergestellte Aminosäure L-Serin Verwendung als Vorstufe für die Synthese von L-Glycin, L-Cystein und/oder L-Tryptophan und/oder davon ableitbaren Stoffwechselprodukten.

Nachfolgend wird die vorliegenden Erfindung durch Beispiele näher erläutert, die jedoch nicht limitierend sind:

### Allgemeine Techniken:

Die Isolierung von Plasmid-DNA aus *Escherichia coli* sowie alle Techniken zur Restriktion, Klenow- und alkalische Phosphatasebehandlung wurden nach Sambrook et al. (Molecular cloning. A laboratory manual (1989) Cold Spring Harbour Laboratory Press) durchgeführt. Die Transformation von *Escherichia coli* wurde, wenn nicht anders beschrieben, nach Chung et al. (Proceedings of the National Academy of Sciences of the United States of America USA (1989) 86: 2172-2175) durchgeführt.

### Klonierung und Sequenzierung des serB-, und serC-Gens von Corynebacterium glutamicum ATCC 14752

### 1. Transposonmutagenese

Der Stamm *Corynebacterium glutamicum* ATCC 14752 wurde einer Mutagenese mit dem Transposon Tn5531 unterworfen, dessen Sequenz unter der Accession-Nummer U53587 in der Nukleotid-Datenbank des National Center for Biotechnology Information (Bethesda, USA) hinterlegt ist. Aus dem Methylase-defekten *Escherichia coli*-Stamm GM2929pCGL0040 *(Escherichia coli* GM2929: Palmer et al., Gene (1994) 143: 1-12) wurde das Plasmid pCGL0040 isoliert, welches das zusammengesetzte Transposon Tn5531 enthält (Ankri et al., Journal of Bacteriology (1996) 178: 4412-4419). Der Stamm *Corynebacterium glutamicum* ATCC 14752 wurde mittels Elektroporation (Haynes et al., FEMS Microbiology Letters (1989) 61: 329-334) mit dem Plasmid pCGL0040 transformiert. Klone, bei denen das Transposon Tn5531 ins Genom integriert war, wurden anhand ihrer Kanamycinresistenz auf 15 µg/mL Kanamycin enthaltenden LBHIS-Agarplatten identifiziert (Liebl et al., FEMS Microbiology Letters (1989) 65: 299-304). Auf diese Weise wurden 1800 Klone erhalten, welche auf verzögertes Wachstum in Anwesenheit von Seryl-Alanin überprüft wurden. Dazu wurden alle Klone einzeln auf CGXII-Minimalmedium-Agarplatten mit und ohne 2 mM Seryl-Alanin übertragen. Das Medium war identisch mit dem bei Keilhauer et al. beschriebenen Medium CGXII (Journal of Bacteriology (1993) 175: 5593-5603), enthielt aber zusätzlich 25 µg/mL Kanamycin und 15 g/L Agar. Die Zusammensetzung des von Keilhauer et al. beschriebenen Mediums ist in Tabelle 1 dargestellt.

Die Agarplatten wurden bei 30°C inkubiert und das Wachstum nach 12, 18 und 24 Stunden untersucht. Es wurden zwei Transposonmutanten erhalten, die mit Seryl-Alanin vergleichbar mit dem Ausgangsstamm *Corynebacterium glutamicum* ATCC 14752 wuchsen, in Abwesenheit von Seryl-Alanin aber kein Wachstum zeigten. Auch mit Serin allein wuchsen die Mutanten, so daß gezeigt war, daß es sich um Serin-auxotrophe Mutanten handelt, die einen Defekt im Serin-Stoffwechsel aufweisen müssen. Diese Mutanten wurde als ATCC 14752ser1::Tn5531 und ATCC 14752ser2::Tn5531 bezeichnet.

### 2. Klonierung und Sequenzierung der Insertionsorte von Tn5531 in ATCC 14752ser1::Tn5531 und ATCC 14752ser2::Tn5531

Um die stromaufwärts des Transposons Tn5531 gelegenen Insertionsorte in den beschriebenen Mutanten zu klonieren, wurde zunächst die chromosomale DNA dieser Mutantenstämme wie bei Schwarzer et al. (Bio/Technology (1990) 9: 84-87) beschrieben isoliert und 400 ng davon mit der Restriktionsendonuklease Xbal geschnitten. Der vollständige Restriktionsansatz wurde in den ebenfalls mit Xbal linearisierten Vektor pUC18 (Norander et al., Gene (1983) 26: 101-106) der Firma Roche Diagnostics (Mannheim, Deutschland) ligiert. Mit dem gesamten Ligationsansatz wurde der *Escherichia coli* Stamm DH5αmcr (Grant et al., Proceedings of the National Academy of Sciences of the United States of America USA (1990) 87: 4645-4649) mittels Elektroporation transformiert (Dower et al., Nucleic Acid Research (1988) 16: 6127-6145). Transformanden, bei denen auf dem Vektor pUC18 die Insertionsorte des Transposons Tn5531 kloniert vorlagen, wurden anhand ihrer Carbenicillin-und Kanamycinresistenz auf 50 µg/mL Carbenicillin und 25 µg/mL Kanamycin enthaltenden LB-Agarplatten identifiziert. Aus jeweils drei der Transformanden wurden die Plasmide präpariert und durch Restriktionsanalyse die Größen der klonierten Inserts bestimmt. Die Nukleotidsequenzen der Insertionsorte auf den Plasmiden mit einem ca. 10 kb großen Inserts im Falle von ser1::Tn5531, bzw. 4,5 kb großen Insert im Falle von ser2::Tn5531 wurden nach der Dideoxy-Kettenabbruchmethode von Sanger et al. bestimmt (Proceedings of the National Academy of Sciences of the United States of America USA (1977) 74: 5463-5467). Hierzu wurden jeweils zunächst ca. 600 bp der beiden Inserts ausgehend von folgendem Oligonukleotid-Primer sequenziert: 5'-CGG GTC TAC ACC GCT AGC CCA GG-3'. Mittels Primer-Walking wurden dann jeweils Sequenzverlängerungen durchgeführt, sodaß insgesamt ca. 1,4 kb des Inserts aus ser1::Tn5531, bzw. 1,2 kb des Inserts aus ser2::Tn5531 sequenziert werden konnten. Die Analyse mit dem Programmpaket Lasergene (Biocomputing Software for Windows, DNASTAR, Madison, USA) ergab, daß in beiden Fällen das Transposon in den Beginn eines offenen Leserahmens inseriert war.

Zur Identifizierung der stromabwärts des Transposons gelegenen Insertionsorte wurde die chromosomale DNA der Mutanten mit der Restriktionsendonuklease *Eco*RI geschnitten und in den mit *Eco*RI linearisierten Vektor pUC18 ligiert. Die weitere Klonierung wurde wie oben beschrieben durchgeführt. Die Nukleotidsequenzen der Insertionsorte auf einem der Plasmide ausgehend von ser1::Tn5531 mit einem ca. 6,5 kb großen Insert, bzw auf einem der Plasmide ausgehend von ser2::Tn5531 mit einem ca. 5,0 kb großen Insert wurden nach der Dideoxy-Kettenabbruchmethode von Sanger et al. bestimmt (Proceedings of the National Academy of Sciences of the United States of America USA (1977) 74: 5463-5467). Hierzu wurden ca. 400 bp des Inserts aus ser1::TN5531, bzw. ca. 220 bp des Inserts aus ser2::Tn5531 ausgehend von folgendem Oligonukleotid-Primer sequenziert: 5'-CGG TGC CTT ATC CAT TCA GG-3'.

Die erhaltenen Nukleotidsequenzen wurden mit dem Programmpaket Lasergene (Biocomputing Software for Windows, DNASTAR, Madison, USA) analysiert und zusammengefügt. Die Nukleotidsequenzen sind als SEO-ID-No. 1 und SEQ-ID-No. 3 wiedergegeben. Die Analyse ergab die Identifizierung von jeweils einem offenen Leseraster von 1209 bp für ser1::Tn5531, und das entsprechende Gen wurde als *serB* bezeichnet, und von 1128 bp Länge für ser2::Tn5531, und das entsprechende Gen wurde als *serC*-Gen bezeichnet. Die dazugehörigen Genprodukte umfassen 403 und 376 Aminosäuren und sind als SEQ-ID-No. 2 und SEQ-ID-No. 4 wiedergegeben.

### Klonierung und Sequenzierung der serB-, und serC-Gene aus Corynebacterium glutamicum ATCC 13032

Die Gene *serB*, und *serC* wurden in den *Escherichia coli* Klonierungsvektor pGEM-T (Zhou M-Y, Clark SE und Gomez-Sanchez CE (1995) BioTechniques 19: 34; Kobs G (1995) Promega Notes 55: 28; Promega Cooperation, Madison, USA) kloniert. Die Klonierung wurde in zwei Schritten durchgeführt. Zunächst wurden durch eine Polymerasekettenreaktion (PCR) jeweils die Gene aus *Corynebacterium glutamicum* ATCC 13032 mittels folgender aus SEQ-ID-No. 1 bzw. SEQ-ID-No. 3 abgeleiteter Oligonukleotid-Primer amplifiziert.
*serB*-forward:
*serB*-reverse:
*serC*-forward:
*serC*-reverse:

Die PCR-Reaktion wurde in 30 Zyklen in Gegenwart von 200 µM Deoxynukleotid-triphosphaten (dATP, dCTP, dGTP, dTTP), je 1 µM des entsprechenden Oligonukleotids, 100 ng chromosomaler DNA von *Corynebacterium glutamicum* ATCC 13032, 1/10 Volumen 10-fach Reaktionspuffer und 2,6 Einheiten einer hitzestabilen Taq-/Pwo-DNA-Polymerase-Mischung (Expand High Fidelity PCR System der Firma Roche Diagnostics, Mannheim, Deutschland) in einem Thermocycler (PTC-100, MJ Research, Inc., Watertown, USA) unter folgenden Bedingungen durchgeführt: 94°C für 60 Sekunden, 56°C für 90 Sekunden und 72°C für 2 Minuten.

Das amplifizierte etwa 1,7 kb große *serB*-Fragment sowie das amplifizierte etwa 1,3 kb große *serC*-Fragment wurden dann im folgenden mit Hilfe des Promega PCR Cloning Kits nach Angaben des Herstellers mit dem Vektor pGEM-T ligiert. Mit beiden Ligationsansätzen wurde der *Escherichia coli* Stamm DH5αmcr (Grant et al., Proceedings of the National Academy of Sciences of the United States of America USA (1990) 87: 4645-4649) transformiert. Transformanden wurden anhand ihrer Ampicillinresistenz auf 50 µg/mL Ampicillin enthaltenden LB-Agarplatten identifiziert. Aus jeweils 10 der Transformanden wurden die Plasmide präpariert und durch Restriktionsanalyse auf das Vorhandensein der 1,7 kb bzw. 1,3 kb PCR-Fragmente als Inserts überprüft. Die so entstandenen rekombinanten Plasmide werden im folgenden mit pGEM-T*serB* und pGEM-T*serC* bezeichnet.

Die Ermittlung der Nukleotidsequenzen der 1,7 kb bzw. 1,3 kb PCR-Fragmente in Plasmid pGEM-T*serB*exp bzw. Plasmid pGEM-T*serC*exp wurden nach der Dideoxy-Kettenabbruchmethode von Sanger et al. durchgeführt (Proceedings of the National Academy of Sciences of the United States of America USA (1977) 74: 5463-5467). Hierzu wurden die vollständigen Inserts von pGEM-T*serB* und pGEM-T*serC* mit Hilfe folgender Primer der Firma Roche Diagnostics (Mannheim, Deutschland) sequenziert.
Universal-Primer:
Reverse-Primer:

Die Nukleotidsequenz des Inserts in dem Plasmid pGEM-T*serB* ist als SEQ-ID-No. 5, die des Inserts in dem Plasmid pGEM-T*serC* als SEQ-ID-No. 7 wiedergegeben. Die erhaltene Nukleotidsequenz wurde mit dem Programmpaket Lasergene (Biocomputing Software for Windows, DNASTAR, Madison, USA) analysiert. Die Analyse ergab die Identifizierung von jeweils einem offenen Leseraster von 1209 bp, und 1128 bp Länge. Die entsprechenden Gene wurden als *serB* und *serC* bezeichnet. Die dazugehörigen Genprodukte kodieren für Polypeptide, die 403 bzw. 376 Aminosäuren lang sind, und die als SEQ-ID-No. 6 und SEQ-ID-No. 8 wiedergegeben sind.

### Überexpression der Gene für die Phosphoserin-Phosphatase serB und die Phosphoserin-Aminotransferase serC

Zur Untersuchung der Auswirkung der Überexpression der Gene für die Phosphoserin-Phosphatase *serB* und die Phosphoserin-Aminotransferase *serC* auf die Serin-Produktion wurden die Expressionsvektoren pVWEX1 (Wendisch, V., Dissertation Heinrich-Heine Universität, Düsseldorf, 1997; vermittelt eine Kanamycin-Resistenz) und pVWEX2 (Wendisch, V., Dissertation Heinrich-Heine Universität, Düsseldorf, 1997; vermittelt eine Tetracyclin-Resistenz) verwendet, die eine IPTG-induzierbare Expression (Molecular Cloning, A laboratory manual (1989) Cold Spring Harbour Laboratory Press) erlauben. In den Vektor pVWEX1 wurde das *serB* Gen und in den Vektor pVWEX2 das *serC* Gen promotorlos hinein kloniert. Dazu wurden zunächst folgende Primer synthetisiert:
*serB*-exp-for:
*serB*-exp-rev:
*serC*-exp-for:
serC-exp-rev:

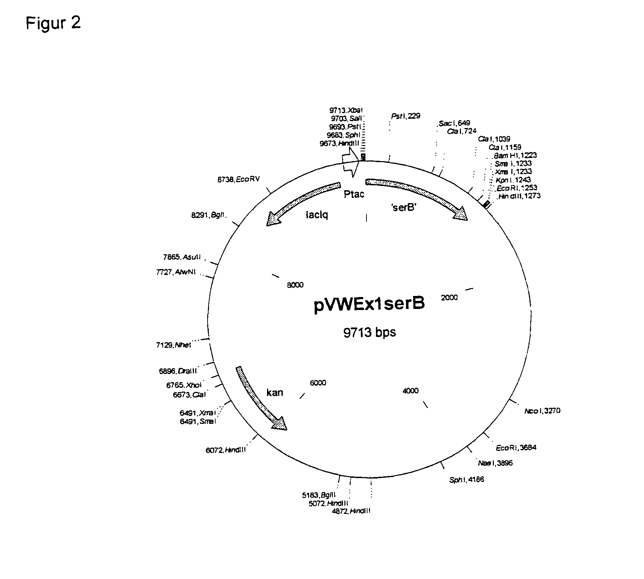

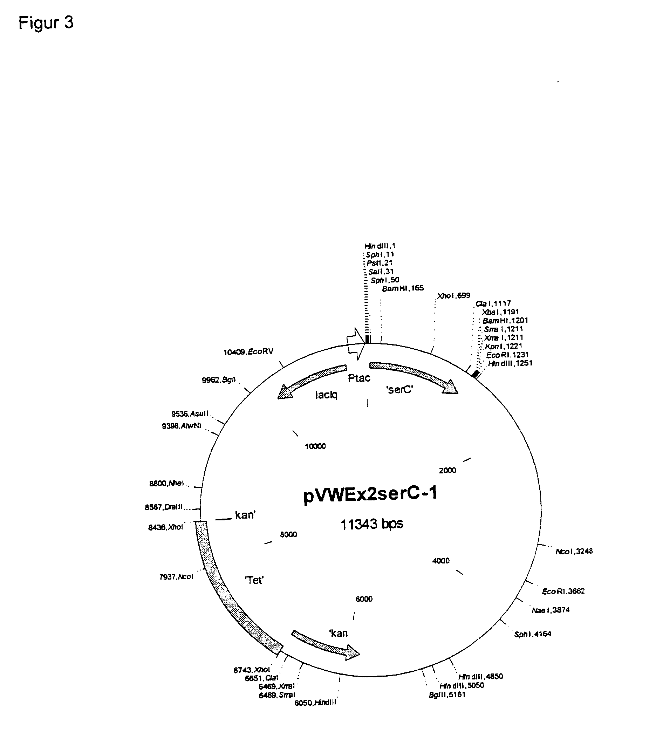

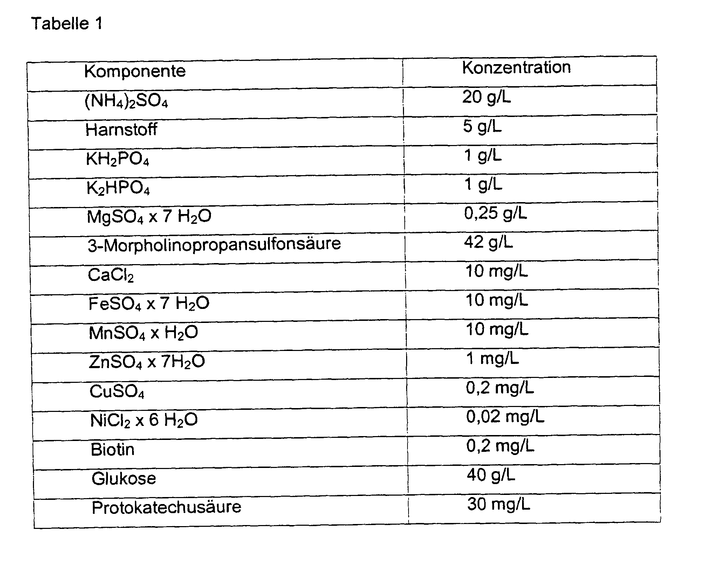

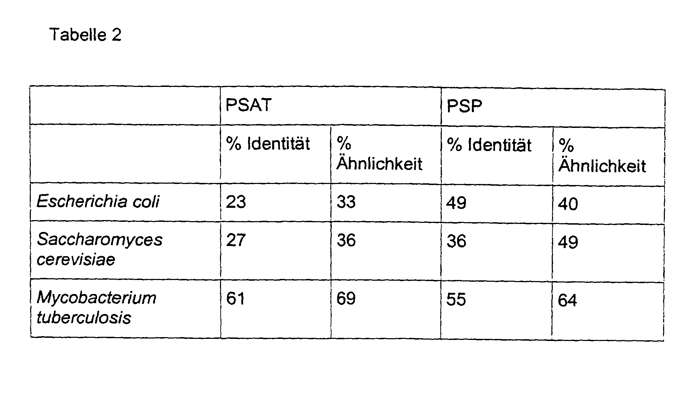

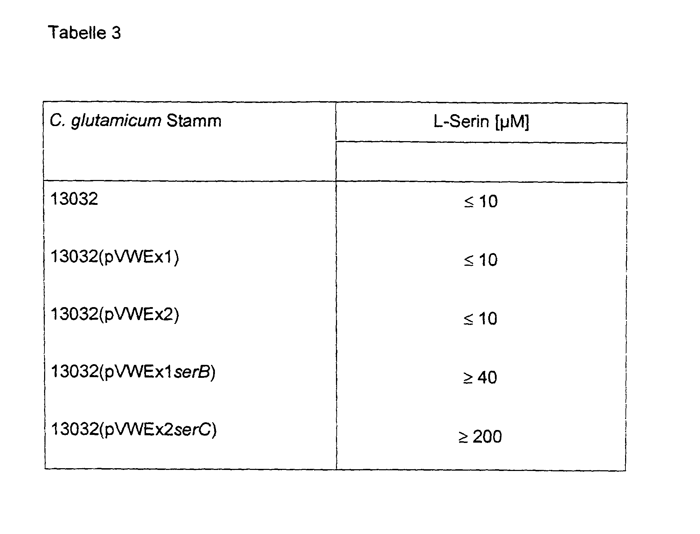

Mittels PCR wurde das promotorlose *serB*-Gen als ein 1226 bp großes Fragment (SEQ ID No. 5 Basen 382 bis 1594) und das promotorlose *serC*-Gen als 1157 bp Fragment (SEQ ID No. 7 Basen 132 bis 1261) aus chromosomaler DNA von *Corynebacterium glutamicum* ATCC 13032 amplifiziert. Die Primer wurden so gewählt, daß Primer *serB*-exp-for eine *Xba*l-Schnittstelle vermittelt, Primer *serB*-exp-rev eine *Bam*HI-Schnittstelle, Primer *serC*-exp-for eine *Sph*I-Schnittstelle und Primer *serC*-exp-rev eine *Xba*l-Schnittstelle. Die isolierten PCR-Produkte wurden zunächst wie oben beschrieben in den Vektor pGEM-T kloniert, und die Plasmide pGEM-T*serB*-exp und pGEM-T*serC*-exp erhalten. Anschließend wurde das promotorlose *serB*-Gen mittels *Xba*I-*Bam*HI-Restriktion aus dem Vektor pGEM-T*serB*exp ausgeschnitten, und in den entsprechend mit *Xba*I-*Bam*HI linearisierten Vektor pVWEX1 ligiert. Das promotorlose *serC*-Gen wurde nach *Spe*I-*Xba*I-Restriktion aus dem Vektor pGEM-T*serB*exp ausgeschnitten und in den mit Xbal linearisierten Vektor pVWEX2 ligiert. Die erhaltenen Konstrukte pVWEX1*serB* (Figur 2) und pVWEX2*serC* (Figur 3) wurden durch Restriktion getestet.

### Gesteigerte Akkumulation von L-Serin durch Überexpression der Gene für die Phosphoserin-Phosphatase serB und die Phosphoserin-Aminotransferase serC

Die Plasmide pVWEX1-*serB* und pVWEX2-*serC* wurden jeweils einzeln durch Elektroporation in den Wildtyp *Corynebacterium glutamicum* Stamm ATCC 13032 eingebracht, resultierend in den Stämmen *C. glutamicum* 13032(pVWEX1*serB*) und *C. glutamicum* 13032(pVWEX2*serC*). Als Negativ-Kontrolle wurde der Wildtyp *Corynebacterium glutamicum* ATCC 13032 sowie der *C. glutamicum*-Stamm ATCC enthaltend den Vektoren pVWEX1 ohne Insert kultiviert. Die L-Serinausscheidung wurde anschließend von allen zuvor genannten Stämmen bestimmt und vergleichend in Tabelle 3 zusammengefaßt.
Dazu wurden die Stämme in Komplexmedium (2xTY; Molecular Cloning, A laboratory manual (1989) Cold Spring Harbour Laboratory Press; mit 50 µg/l Kanamycin, 25 µg/l Tetracyclin bzw. 50 µg/l Kanamycin und 25 µg/l Tetracyclin) gezüchtet, und das Fermentationsmedium CGXII (J Bacteriol (1993) 175: 5595-5603) jeweils aus den Vorkulturen getrennt beimpft. Das Medium enthielt zusätzlich das/die entsprechende(n) Antibiotika sowie 200 µg/ml IPTG. Nach Kultivierung für 24 Stunden bei 30°C auf dem Rotationsschüttler bei 120 Umdrehungen pro Minute wurde die in das Medium akkumulierte L-Serinmenge bestimmt. Die Bestimmung der Aminosäurekonzentration erfolgte mittels Hochdruckflüssigkeitchromatographie (J Chromat (1983) 266: 471-482).

### Legende zu den Sequenzprotokollen, Figuren und Tabellen

### Sequenzprotokoll:

Darstellung der Nukleinsäuresequenzen enthaltend die Gene *serB* (SEQ ID No 1), *serC* (SEQ ID No 3) und *thrE* (SEQ ID No 9) sowie davon abgeleitete Aminosäuresequenzen SerB (SEQ ID No 2), SerC (SEQ ID No 4) und ThrE (SEQ ID No 10) aus *Corynebacterium glutamicum* ATCC 14752 und *Corynebacterium glutamicum* ATCC 13032 (entsprechend SEQ ID No 5, 7, 6, 8, 11 und 12)
Figur 1: Schematische Darstellung des Vektors pCGL0040
   Die Bedeutung der verwendeten Abkürzungen ist wie folgt:
   Amp = β-Lactamase-Gen, welches Ampicillin-Resistenz vermittelt
   Kan = Phosphotransferase-Gen, welches Kanamycin-Resistenz vermittelt
   Ferner sind Schnittstellen für Restriktionsendonukleasen angegeben
Figur 2: Schematische Darstellung des Vektors pVWEx1*serB*
   Die Bedeutung der verwendeten Abkürzungen ist wie folgt:
   - Kan =: Phosphotransferase-Gen, welches Kanamycin-Resistenz vermittelt
   - lacl^{q} =: quantitativ exprimierter Repressor des Lactose-Operons aus *E. coli*
   - P^{tac} =: IPTG-induzierbarer, artifizieller Promotor zusammengesetzt aus dem trp-Promotor und dem lac-Promotor von *E. coli*
   Ferner sind Schnittstellen für Restriktionsendonukleasen angegeben
Figur 3: Schematische Darstellung des Vektors pVWEx1*serC*-1

Die Bedeutung der verwendeten Abkürzungen ist wie folgt:
Kan = Phosphotransferase-Gen, welches Kanamycin-Resistenz vermittelt
   - Tet: = tetα1-Gen aus dem Plasmid pHY163PLK (Ishiwa & Shibahara, 1985, Jpn. J. Genet., 60:485-498), welches Tetracyclin-Resistenz vermittelt
   - lacl^{q}: = quantitativ exprimierter Repressor des Lactose-Operons aus *E. coli*
   - P^{tac} =: IPTG-induzierbarer, artifizieller Promotor zusammengesetzt aus dem trp-Promotor und dem lac-Promotor von *E. coli* Ferner sind Schnittstellen für Restriktionsendonukleasen angegeben
   - Tabelle 1:: Zusammensetzung des Mineralsalz-Mediums CGXII zur Kultivierung von coryneformen Bakterien
   - Tabelle 2:: Vergleich der Ähnlichkeiten der Phosphoserin-Aminotransferase (PSAT; *serC*) und der Phosphoserin-Phosphatase (PSP; *serB*) von *Corynebacterium glutamicum* zu bekannten Phosphoserin-Aminotransferasen und Phosphoserin-Phosphatasen anderer Organismen
   - Tabelle 3:: Vergleichende Übersicht der Akkumulation von L-Serin im Kulturüberstand des *Corynebacterium glutamicum* Wildtyps ATCC 13032 sowie der mit den entsprechenden Plasmiden transformierten *Corynebacterium glutamicum* Stämme ATCC 13032(pVWEX1), ATCC 13032(pVWEX1*serB*) und ATCC 13032(pVWEX2*serC*)

## Patentansprüche

1. Verfahren zur mikrobiellen Herstellung von L-Serin, **dadurch gekennzeichnet, daß**
a) eine Nukleinsäure kodierend für eine Phosphoserin-Phosphatase (serB) gemäß SEQ ID No. 1 oder 5 und eine Nukleinsäure kodierend für eine Phosphoserin-Aminotransferase (serC) gemäß SEQ ID No 3 oder 7 und eine Nukleinsäure kodierend für einen L-Threoninexportcarrier gemäß SEQ ID No 9 oder 11 isoliert aus einem coryneformen Bakterium in einen homologen Mikroorganismus übertragen und dort exprimiert werden, wobei die Expression und die Aktivität der entsprechend kodierten Polypeptide gegenüber dem entsprechend nicht genetisch veränderten Mikroorganismus erhöht sind,
b) dieser genetisch veränderte Mikroorganismus aus Schritt a) zur fermentativen Herstellung von L-Serin eingesetzt wird, wobei L-Serin verstärkt in das Kulturmedium sezemiert wird und
c) das entsprechend gebildete L-Serin aus dem Kulturmedium isoliert wird.

2. Genetisch veränderter Mikroorganismus zur Herstellung von L-Serin enthaltend in replizierbarer Form eine Nukleinsäure kodierend für eine Phosphoserin-Phosphatase (serB) gemäß SEQ ID No. 1 oder 5 und eine Nukleinsäure kodierend für eine Phosphoserin-Aminotransferase (serC) gemäß SEQ ID No 3 oder 7 und eine Nukleinsäure kodierend für den L-Threoninexportcarrier (thrE) gemäß SEQ ID No 9 oder 11, welche im Vergleich zu dem entsprechend nicht genetisch veränderten Mikroorganismus verstärkt exprimiert werden und/oder deren Kopienzahl erhöht ist.

3. Genetisch veränderter Mikroorganismus gemäß Anspruch 2, enthaltend Polypeptide kodiert durch die Gene serB, serC und thrE, welche eine erhöhte Aktivität im Vergleich zu dem entsprechend nicht genetisch veränderten Mikroorganismus aufweisen.

4. Genetisch veränderter Mikroorganismus gemäß einem der Ansprüche 2 oder 3, **dadurch gekennzeichnet, daß** er wenigstens eine erhöhte L-Serinproduktionsrate und eine erhöhte L-Serin- und/oder L-Threonin-Sekretionsrate aufweist.

5. Genetisch veränderter Mikroorganismus gemäß einem der Ansprüche 2 bis 4, **dadurch gekennzeichnet, daß** er ein coryneformes Bakterium ist, bevorzugt der Gattung *Corynebacterium* oder *Brevibacterium*.

6. Genetisch veränderter Mikroorganismus gemäß einem der Ansprüche 2 bis 5, **dadurch gekennzeichnet, daß** er ein Bakterium der Spezies *Corynebacterium glutamicum* oder *Brevibacterium flavum* ist.

7. Verwendung der Nukleinsäuren gemäß SEQ ID No. 1, 3, 5 oder 7 und SEQ ID No. 9 oder 11 zur Herstellung eines genetisch veränderten Mikroorganismus gemäß einem der Ansprüche 2-6.

8. Verwendung der Sequenzen gemäß SEQ ID No. 9-12 zum verbesserten Export von L-Serin in einem genetisch veränderten Mikroorganismus gemäß einem der Ansprüche 2-6.

9. Verwendung des genetisch veränderten Mikroorganismus gemäß einem der Ansprüche 2 bis 6 zur Herstellung von L-Serin und/oder deren Folgeprodukte.

## Claims

1. Method for the microbial production of L-serine,
**characterized in that**
a) a nucleic acid coding for a phosphoserine phosphatase (serB) according to SEQ ID No. 1 or 5 and a nucleic acid coding for a phosphoserine aminotransferase (serC) according to SEQ ID No. 3 or 7 and a nucleic acid coding for an L-threonine export carrier according to SEQ ID No. 9 or 11, isolated from a coryneform bacterium, are transferred into a homologous microorganism and expressed therein, the expression and the activity of the correspondingly encoded polypeptides being increased compared to the correspondingly genetically unmodified microorganism,
b) this genetically modified microorganism from step a) is used for the fermentative production of L-serine, L-serine being increasingly secreted into the culture medium, and
c) the L-serine correspondingly formed is isolated from the culture medium.

2. Genetically modified microorganism for producing L-serine, comprising, in a replicable form, a nucleic acid coding for a phosphoserine phosphatase (serB) according to SEQ ID No. 1 or 5 and a nucleic acid coding for a phosphoserine aminotransferase (serC) according to SEQ ID No. 3 or 7 and a nucleic acid coding for the L-threonine export carrier (thrE) according to SEQ ID No. 9 or 11, whose expression is enhanced and/or whose copy number is increased, compared to the correspondingly genetically unmodified microorganism.

3. Genetically modified microorganism according to Claim 2, comprising polypeptides encoded by the genes serB, serC and thrE, which have an increased activity compared to the correspondingly genetically unmodified microorganism.

4. Genetically modified microorganism according to either of Claims 2 and 3, **characterized in that** it has at least an increased rate of L-serine production and an increased rate of L-serine and/or L-threonine secretion.

5. Genetically modified microorganism according to any of Claims 2 to 4, **characterized in that** it is a coryneform bacterium, preferably of the genus *Corynebacterium* or *Brevibacterium.*

6. Genetically modified microorganism according to any of Claims 2 to 5, **characterized in that** it is a bacterium of the species *Corynebacterium glutamicum* or *Brevibacterium flavum*.

7. Use of nucleic acids according to SEQ ID No. 1, 3, 5 or 7 and SEQ ID No. 9 or 11 for producing a genetically modified microorganism according to any one of Claims 2-6.

8. Use of sequences according to SEQ ID No. 9-12 for the improved export of L-serine in a genetically modified microorganism according to any one of Claims 2-6.

9. Use of the genetically modified microorganism according to any of Claims 2 to 6 for producing L-serine and/or secondary products thereof.

## Revendications

1. Procédé de préparation microbienne de L-sérine, **caractérisé en ce que**
a) un acide nucléique codant pour une phosphosérine-phosphatase (serB) selon la SEQ ID N° 1 ou 5 et un acide nucléique codant pour une phosphosérine-aminotransférase (serC) selon la SEQ ID N° 3 ou 7 et un acide nucléique codant pour un support d'exportation de L-thréonine selon la SEQ ID N° 9 ou 11 isolé d'une bactérie coryneforme sont transférés dans un microorganisme homologue et y sont exprimés, l'expression et l'activité des polypeptides codés de manière correspondante étant plus élevées par rapport au microorganisme non génétiquement modifié de manière correspondante,
b) ce microorganisme génétiquement modifié de l'étape a) est utilisé pour la préparation fermentative de L-sérine, dans laquelle la L-sérine est secrétée de manière amplifiée dans le milieu de culture, et
c) la L-sérine formée de manière correspondante est isolée du milieu de culture.

2. Microorganisme génétiquement modifié pour la préparation de L-sérine contenant sous forme réplicable un acide nucléique codant pour une phosphosérine-phosphatase (serB) selon la SEQ ID N° 1 ou 5 et un acide nucléique codant pour une phosphosérine-aminotransférase (serC) selon la SEQ ID N° 3 ou 7 et un acide nucléique codant pour le support d'exportation de L-thréonine (thrE) selon la SEQ ID N° 9 ou 11, qui sont exprimés de manière amplifiée en comparaison du microorganisme non génétiquement modifié de manière correspondante et/ou dont le nombre de copies est accru.

3. Microorganisme génétiquement modifié selon la revendication 2, contenant des polypeptides codés par les gènes serB, serC et thrE, qui présentent une activité plus élevée en comparaison du microorganisme non génétiquement modifié de manière correspondante.

4. Microorganisme génétiquement modifié selon l'une quelconque des revendications 2 ou 3, **caractérisé en ce qu'**il présente au moins un taux de production de L-sérine plus élevé et un taux de sécrétion de L-sérine et/ou de L-thréonine plus élevé.

5. Microorganisme génétiquement modifié selon l'une quelconque des revendications 2 à 4, **caractérisé en ce qu'**il s'agit d'une bactérie coryneforme, de préférence de la souche *Corynebacterium* ou *Brevibacterium*.

6. Microorganisme génétiquement modifié selon l'une quelconque des revendications 2 à 5, **caractérisé en ce qu'**il s'agit d'une bactérie de la souche *Corynebacterium glutamicum* ou *Brevibacterium flavum*.

7. Utilisation des acides nucléiques selon la SEQ ID N° 1, 3, 5 ou 7 et la SEQ ID N° 9 ou 11 pour la préparation d'un microorganisme génétiquement modifié selon l'une quelconque des revendications 2 à 6.

8. Utilisation des séquences selon les SEQ ID N° 9 à 12 pour l'exportation améliorée de L-sérine dans un microorganisme génétiquement modifié selon l'une quelconque des revendications 2 à 6.

9. Utilisation du microorganisme génétiquement modifié selon l'une quelconque des revendications 2 à 6 pour la préparation de L-sérine et/ou de ses produits dérivés.
